# FASCICULE DE BREVET EUROPEEN

(11) **EP 1 349 850 B1**
(45) Date de publication et mention de la délivrance du brevet: **07.09.2005**
(21) Numéro de dépôt: 01989665.3
(22) Date de dépôt: 27.12.2001
(51) Int. Cl.: C07D 401/04, A61K 31/445, A61K 31/415

(54) **MODULATEURS DE CANAUX SODIQUES DERIVES DE 2-PIPERIDYLIMIDAZOLES**
2-PIPERIDYLIMIDAZOL-DERIVATE ALS MODULATOREN DER NA-KANÄLE
SODIUM CHANNEL MODULATORS DERIVED FROM 2-PIPERIDYLIMIDAZOLES

(30) Priorité: 28.12.2000 FR 0017149
(43) Date de publication de la demande: 08.10.2003
(73) Titulaire: Société de Conseils de Recherches et d'Applications Scientifiques ( S.C.R.A.S.), 75016 Paris (FR)
(72) Inventeur: BIGG, Dennis, F-91190 Gif-sur-Yvette (FR); POMMIER, Jacques, F-75015 Paris (FR); LIBERATORE, Anne-Marie, F-78610 Auffargis (FR)
(74) Mandataire: Bourgouin, André
(86) Numéro de dépôt international: PCT/FR2001/004209
(87) Numéro de publication internationale: WO 2002/053559

(56) Documents cités:
- WO-A-00/77008
- WO-A-93/20061
- WO-A-97/12876
- WO-A-97/47618
- WO-A-99/64420
- US-A- 5 378 847

## Description

La présente invention concerne de nouveaux modulateurs de canaux sodiques dérivés de 2-pipéridylimidazoles.

Les composés modulateurs de canaux sodiques sont très utiles pour des utilisations thérapeutiques comme :
- le traitement ou la prévention de la douleur, et notamment :
   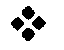 des douleurs neuropathiques telles que la névralgie du trijumeau, la douleur post-herpétique, les neuropathies diabétiques, les névralgies glosso-pharyngiennes, les radiculopathies et neuropathies secondaires à des infiltrations métastatiques, l'adiposis dolorosa et les douleurs liées aux brûlures,
   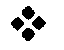 de la migraine,
   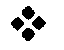 des douleurs post-opératoires,
   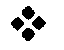 des douleurs centrales consécutives aux accidents cérébraux vasculaires, lésions thalamiques et sclérose en plaques, et
   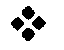 des douleurs chroniques inflammatoires ou liées à un cancer ;
- le traitement de l'épilepsie ;
- le traitement des troubles du rythme cardiaque ;
- le traitement de troubles liés à la neurodégénération, et en particulier :
   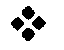 des accidents cérébraux vasculaires,
   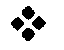 du traumatisme cérébral, et
   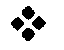 de maladies neurodégénératives telles que la maladie d'Alzheimer, la maladie de Parkinson et la sclérose latérale amyotrophique ;
- le traitement de la dépression et des troubles bipolaires ;
- le traitement du syndrome du colon irritable ;
- le traitement des rétinopathies diabétiques.

La demanderesse avait déjà décrit, dans une demande antérieure non publiée à la date de la présente demande, des composés modulateurs des canaux sodiques répondant à la formule générale 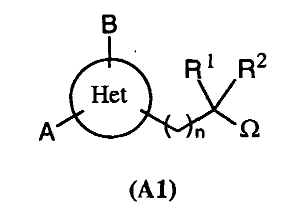 sous forme racémique, d'énantiomère ou toute combinaison de ces formes, dans laquelle Het est un hétérocycle à 5 chaînons comportant 2 hétéroatomes et tel que la formule générale **(A1)** corresponde exclusivement à l'une des sous-formules suivantes : 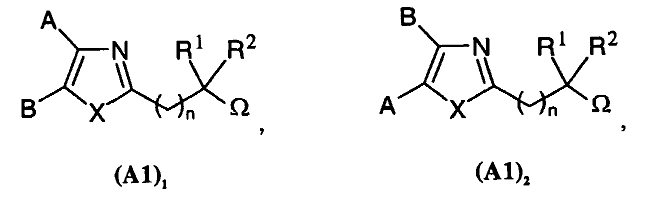 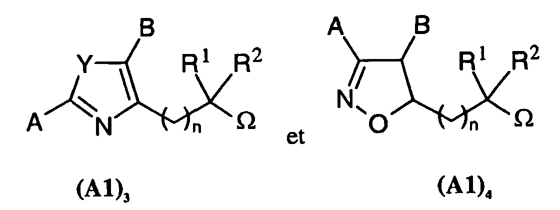 dans lesquelles
A représente notamment un radical alkyle, cycloalkyle ou phényle éventuellement substitué ;
B représente notamment un atome d'hydrogène ou un radical alkyle, cycloalkyle ou phényle éventuellement substitué ;
X représente notamment NH ou S ;
Y représente O ou S ;
R¹ et R² représentent notamment indépendamment un atome d'hydrogène, un radical alkyle ou cycloalkyle ;
Ω représente l'un des radicaux NR⁴⁶R⁴⁷ ou OR⁴⁸ dans lesquels R⁴⁶ et R⁴⁷ représentent notamment un atome d'hydrogène, un radical alkyle, cycloalkyle, cycloalkylalkyle ou aralkyle ou encore un radical -COOR⁵¹ dans lequel R⁵¹ représente notamment un radical alkyle, aryle ou aralkyle.

Un certain nombre de brevets ou demandes de brevet décrit par ailleurs des dérivés de 2-pipéridylimidazoles.

La demande de brevet PCT WO 96/16040 décrit les composés de formule générale **(A2)** 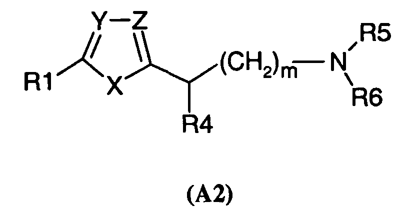 dans laquelle
R1 représente l'un des radicaux aryle, hétéroaryle, aralkyle ou cycloalkyle optionnellement substitués par un à trois substituants choisis indépendamment parmi un atome halogène, le radical CF₃, CN, OH, alkyle ou alkoxy, SO₂R9 avec R9 représentant NH₂ ou NHCH₃;
X représente NR2, R2 représentant H ou alkyle ;
Y représente N ou CR3 ;
Z représente CR3 ou N ;
à la condition toutefois que Y et Z ne sont pas tous deux CR3 ou N en même temps ;
R3 représente H, alkyle, halogène, hydroxyalkyle ou phényle éventuellement substitué par 1 à 3 subtituants choisis parmi H, CF₃, CN, SO₂NH₂, OH, alkyle ou alkoxy ;
m représente 0, 1 ou 2 ;
R4 représente H ou alkyle ;
lorsque Z représente CR3, alors R3 et R4 peuvent aussi représenter ensemble -(CH₂)ₙ₁- avec n1 entier de 2 à 4 ou R2 et R4 peuvent aussi représenter ensemble -(CH₂)ₙ₂- avec n2 entier de 2 à 4 ;
R5 et R6 représentent indépendamment H, alkyle, alkoxy, aryle ou aralkyle ;
R4 et R5 pouvant par ailleurs représenter ensemble -(CH₂)ₙ₃- avec n3 représentant 2 ou 3;
NR5R6 pouvant aussi représenter ensemble (notamment) :
- le radical 2-(1,2,3,4-tétrahydroquinolyl) éventuellement substitué,
- un radical 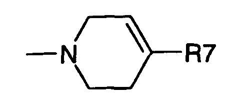
dans lequel R7 représente l'un des radicaux phényle, benzyle ou phénéthyle dans lequels le cycle phényle peut être substitué ;
- un radical 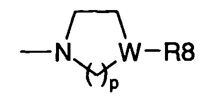
dans lequel p est un entier de 1 à 3,
W est N et R8 représente H, CF₃, l'un des radicaux phényle, pyridyle ou pyrimidinyle éventuellement substitués de 1 à 2 fois par des radicaux choisis parmi halogène, OH, alkyle ou alkoxy, ou
W est CH et R8 représente phényle éventuellement substitué ou aralkyle éventuellement substitué sur le groupe aryle ;
ces composés étant agonistes partiels ou antagonistes des sous-récepteurs de la dopamine du cerveau ou des formes prodrogues de tels agonistes partiels ou antagonistes. Des propriétés intéressantes seraient de ce fait obtenues dans le diagnostic et le traitement de désordres affectifs tels que la schizophrénie et la dépression ainsi que certains désordres du mouvement tels que la maladie de Parkinson.

Les brevets américains 5,717,100 et 6,083,349 et les demandes PCT WO 97/12876, WO 97/36587 et WO 97/47618 indiquent principalement que les composés de formule générale **(A3)** 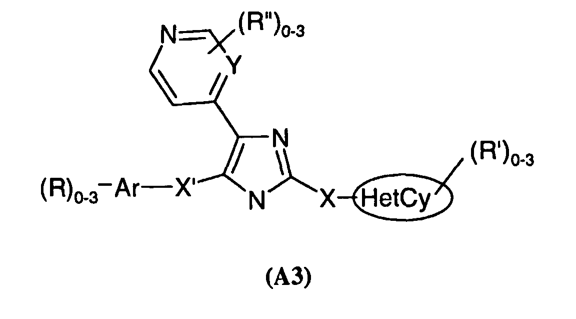 dans laquelle :
Ar représente un radical aryle éventuellement substitué par un à trois substituants choisis indépendamment parmi les substituants R ;
X et X' représentent notamment un radical -(CH₂)ₘ-Y-(CH₂)ₙ- dans lequel m et n sont des entiers de 0 à 4 et Y représente notamment une liaison, O, S, SO, SO₂, OCO, COONH ou CONH ;
Y représente CH ou N ;
HetCy représente un hétérocycle non aromatique de 4 à 10 chaînons comportant au moins un atome d'azote ;
les radicaux R et R•, chaque fois qu'ils interviennent, sont choisis indépendamment parmi le groupe comprenant notamment un atome halogène, un groupe OH, CF₃, SH, NH₂ ou NO₂ et un radical alkyle éventuellement substitué, hétérocyclyle ou hétéroaryle ;
et le radical R' est, chaque fois qu'il intervient, choisi indépendamment parmi le groupe comprenant notamment les radicaux hydroxy, amino, alkyle éventuellement substitué, hétérocyclyle et hétéroaryle ;
peuvent être utilisés comme agents anti-cancéreux.

Outre les documents précités, la demande de brevet PCT WO 00/57877 décrit notamment les composés de formule générale **(A4)** 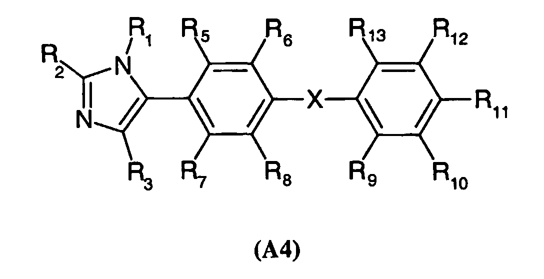 dans laquelle
R₁ représente (notamment) un atome d'hydrogène ou un radical alkyle,
R₂ et R₃ représentent indépendamment un atome d'hydrogène ou un radical alkyle, cycloalkyle, alkényle, alkynyle, haloalkyle, aryle, aminoalkyle, hydroxyalkyle, alkoxyalkyle, alkylthio, alkylsulfinyle, alkylsulfonyle, carboxyalkyle, cyano, amino, alkylamino, aminocarbonyle, alkylaminocarbonyle, arylaminocarbonyle, aralkylaminocarbonyle, alkylcarbonylamino, arylcarbonylamino, aralkylcarbonylamino, alkylcarbonyle, hétérocyclocarbonyle, aminosulfonyle, alkylaminosulfonyle et hétérocyclosulfonyle,
X représente O, S ou un radical NR₁₅ dans lequel R₁₅ représente un atome d'hydrogène, un radical alkyle ou cycloalkyle,
et R₅, R₆, R₇, R₈, R₉, R₁₀, R₁₁, R₁₂ et R₁₃ représentent (notamment) indépendamment un atome d'hydrogène, un atome halogène et un radical alkyle, hydroxy ou alkoxy,
en tant qu'inhibiteurs des canaux sodiques.

La demanderesse vient à présent de découvrir une nouvelle classe de composés modulant les canaux sodiques, à savoir les dérivés de 2-pipéridylimidazoles décrits ci-après.

Selon l'invention, les composés répondant à la formule générale **(I)** 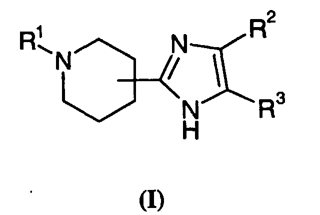 sous forme racémique, d'énantiomère ou toute combinaison de ces formes, dans laquelle :
R¹ représente un atome d'hydrogène ou un radical aralkyle ou cycloalkylalkyle ou encore R¹ représente un radical -X-Y-R⁴ dans lequel le groupe -X-Y- représente -CO-, -CO-O-, -CO-NH- ou -CS-NH- et R⁴ représente un radical alkyle, haloalkyle, cycloalkyle, cycloalkylalkyle, aryle ou aralkyle ;
R² représente un radical phényle substitué par un atome halogène, un radical alkyle ou alkoxy, un radical NR⁵R⁶ ou un radical phényle lui-même éventuellement substitué par un atome halogène ou un radical alkoxy,
R⁵ et R⁶ représentant indépendamment un atome d'hydrogène ou un radical alkyle ou R⁵ et R⁶ formant ensemble avec l'atome d'azote déjà présent un hétérocycle de 5 à 7 chaînons dont les chaînons complémentaires sont choisis parmi -CH₂-, -O-, -S- et -NR⁸-,
R⁸ représentant indépendamment à chaque fois qu'il intervient un atome d'hydrogène ou un radical alkyle, haloalkyle, cycloalkyle, cycloalkylalkyle, aryle ou aralkyle, lesdits radicaux aralkyle ou aryle étant éventuellement substitués de 1 à 4 fois sur le radical aryle par un ou des radicaux choisis parmi un atome halogène et un radical alkyle, hydroxy ou alkoxy ;
R³ représente un atome d'hydrogène,
étant entendu que, dans les définitions qui précèdent :
- chaque radical alkyle ou alkoxy est linéaire ou ramifié et compte de 1 à 12 atomes de carbone:
   - chaque radical haloalkyle est un radical alkyle dont au moins l'un des atomes d'hydrogène est remplacé par un atome halogène
   - chaque radical aryle est choisi parmi les radicaux phényle, naphtyle et phénantryle ; et
   - chaque radical cycloalkyle compte de 3 à 7 atomes de carbone ;
ou les sels pharmaceutiquement acceptables de ces composés ;
peuvent être utilisés pour préparer un médicament destiné à avoir une activité modulatrice vis-à-vis des canaux sodiques.

Par halogène, on entend les atomes de fluor, de chlore, de brome ou d'iode.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", *Int. J. Pharm*. (1986), **33,** 201-217.

De préférence, les composés de formule générale **(I)** définie ci-dessus (ou leurs sels pharmaceutiquement acceptables) possèderont au moins l'une des caractéristiques suivantes :
- le cycle pipéridyle des composés de formule générale **(I)** est substitué en position 3 ou 4 par le radical imidazolyle portant les groupes R² et R³ ;
- R¹ représente un atome d'hydrogène ou un radical aralkyle ou cycloalkylalkyle ou encore R¹ représente un radical -X-Y-R⁴ dans lequel le groupe -X-Y- représente -CO-, -CO-O-, -CO-NH- ou -CS-NH- et R⁴ représente un radical alkyle, haloalkyle, cycloalkyle, cycloalkylalkyle, aryle ou aralkyle ;
- R² représente un radical phényle substitué par un atome halogène, un radical alkyle ou un radical phényle lui-même éventuellement substitué par un atome halogène ;
- R³ représente un atome d'hydrogène.

Plus préférentiellement, les composés de formule générale **(I)** définie ci-dessus (ou leurs sels pharmaceutiquement acceptables) possèderont au moins l'une des caractéristiques suivantes :
- le cycle pipéridyle des composés de formule générale **(I)** est substitué en position 3 ou 4 par le radical imidazolyle portant les groupes R² et R³ ;
- R¹ représente un atome d'hydrogène ou un radical benzyle ou cyclohexylalkyle ou encore R¹ représente un radical -X-Y-R⁴ dans lequel le groupe -X-Y- représente -CO-, -CO-O-, -CO-NH- ou -CS-NH- et R⁴ représente un radical alkyle ou aralkyle ;
- R² représente un radical phényle substitué par un atome halogène, un radical alkyle ou alkoxy ou un radical phényle lui-même éventuellement substitué par un atome halogène ;
- R³ représente un atome d'hydrogène.

Bien que l'on préfère les cas où le cycle pipéridyle des composés de formule générale **(I)** est substitué en position 3 ou 4 par le radical imidazolyle portant les groupes R² et R³, une autre variante intéressante est constituée par les cas où le cycle pipéridyle des composés de formule générale **(I)** est substitué en position 2 par le radical imidazolyle portant les groupes R² et R³.

Selon une variante préférée de l'invention, les composés de formule générale **(I)** définie ci-dessus (ou leurs sels pharmaceutiquement acceptables) seront tels que le radical R¹ représente un radical -X-Y-R⁴ dans lequel :
- soit -X-Y- représente une liaison et R⁴ représente un radical cycloalkyle, cycloalkylalkyle ou aralkyle, ledit radical aralkyle étant éventuellement substitué de 1 à 4 fois (de préférence de 1 à 3 fois et plus préférentiellement de 1 à 2 fois) sur le radical aryle par un ou des radicaux choisis parmi un atome halogène et un radical alkyle, hydroxy ou alkoxy ;
- soit -X-Y- représente un radical -CO-, -CO-O-, -CO-NH- ou -CS-NH- et R⁴ représente un radical alkyle, haloalkyle, cycloalkyle, cycloalkylalkyle, aryle ou aralkyle, lesdits radicaux aralkyle ou aryle étant éventuellement substitués de 1 à 4 fois (de préférence de 1 à 3 fois et plus préférentiellement de 1 à 2 fois) sur le radical aryle par un ou des radicaux choisis parmi un atome halogène et un radical alkyle, hydroxy ou alkoxy.

Selon une autre variante préférée de l'invention, les composés de formule générale **(I)** définie ci-dessus (ou leurs sels pharmaceutiquement acceptables) seront tels que le radical R² représente un radical phényle substitué par un radical phényle lui-même éventuellement substitué par un atome halogène ou un radical alkoxy.

Selon encore une autre variante préférée de l'invention, les composés de formule générale **(I)** dans lesquels l'un au moins de R¹, R² et R³ est un groupe piégeur de radicaux libres (ou leurs sels pharmaceutiquement acceptables) pourront être utilisés pour préparer un médicament spécialement destiné à moduler les canaux sodiques et à piéger les espèces réactives de l'oxygène (ou ROS pour *Reactive Oxygen Species*). Pour cette variante préférée, les mêmes préférences que celles indiquées précédemment de façon générale pour les composés de formule générale **(I)** sont applicables *mutatis mutandis.*

Seront particulièrement préférés pour une utilisation selon l'invention les composés décrits (parfois sous forme de sels) dans les exemples 1 à 26. Encore plus préférentiellement, les composés de l'invention seront choisis parmi les composés des exemples 1 à 9, 12 à 14, 17, 19 à 24 et 26 décrits ci-après.

L'invention concerne par ailleurs l'utilisation d'un composé de formule générale **(I)** telle que définie précédemment ou un sel pharmaceutiquement acceptable d'un tel composé pour préparer un médicament destiné à avoir une activité modulatrice des canaux sodiques chez le patient auquel il aura été administré. On préférera en particulier utiliser un composé de formule générale **(I)** telle que définie précédemment ou un sel pharmaceutiquement acceptable d'un tel composé pour préparer un médicament destiné à traiter la douleur, notamment les douleurs neuropathiques et la migraine.

L'invention a de plus pour objet, à titre de médicaments, les composés de formule générale **(I)** répondant à la sous-formule générale **(I)**_{**M**}**.** 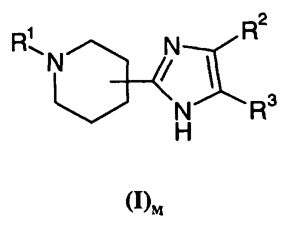 sous forme racémique, d'énantiomère ou toute combinaison de ces formes, dans laquelle :
R¹ représente un radical aralkyle ou cycloalkylalkyle ou encore R¹ représente un radical -X-Y-R⁴ dans lequel le groupe -X-Y- représente -CO-, -CO-O-, -CO-NH- ou -CS-NH- et R⁴ représente un radical alkyle, haloalkyle, cycloalkyle, cycloalkylalkyle, aryle ou aralkyle ;
R² représente un radical phényle substitué par un atome halogène, un radical alkyle ou alkoxy, un radical NR⁵R⁶ ou un radical phényle lui-même éventuellement substitué par un atome halogène ou un radical alkoxy,
R⁵ et R⁶ représentant indépendamment un atome d'hydrogène ou un radical alkyle ou R⁵ et R⁶ formant ensemble avec l'atome d'azote déjà présent un hétérocycle de 5 à 7 chaînons dont les chaînons complémentaires sont choisis parmi -CH₂-, -O-, -S- et -NR⁸-,
R⁸ représentant indépendamment à chaque fois qu'il intervient un atome d'hydrogène ou un radical alkyle, haloalkyle, cycloalkyle, cycloalkylalkyle, aryle ou aralkyle, lesdits radicaux aralkyle ou aryle étant éventuellement substitués de 1 à 4 fois sur le radical aryle par un ou des radicaux choisis parmi un atome halogène et un radical alkyle, hydroxy ou alkoxy ;
R³ représente un atome d'hydrogène
étant entendu que, dans les définitions qui précèdent :
- chaque radical alkyle ou alkoxy est linéaire ou ramifié et compte de 1 à 12 atomes de carbone ;
- chaque radical haloalkyle est un radical alkyle dont au moins l'un des atomes d'hydrogène est remplacé par un atome halogène
- chaque radical aryle est choisi parmi les radicaux phényle, naphtyle et phénantryle ; et
- chaque radical cycloalkyle compte de 3 à 7 atomes de carbone ;
ou les sels pharmaceutiquement acceptables des composés de formule générale **(I)**_{**M**}**.**

L'invention a de plus pour objet, à titre de médicaments, les composés décrits (parfois sous forme de sels) dans les exemples 1 à 19 et leurs sels pharmaceutiquement acceptables.

L'invention a aussi pour objet, en tant que produits nouveaux, les composés de formule générale **(I)**_{**M**} telle que définie précédemment ou leurs sels. Elle concerne également, en tant que produits nouveaux, les composés décrits dans les exemples 1 à 26 (parfois sous forme de sels) et leurs sels.

L'invention concerne de plus les compositions pharmaceutiques comprenant, à titre de principe actif, au moins un composé de formule générale **(I)**_{**M**} telle que définie précédemment ou un sel pharmaceutiquement acceptable d'un tel composé. Elle a aussi pour objet les compositions pharmaceutiques comprenant, à titre de principe actif, au moins un composé décrit dans les exemples 1 à 26 (parfois sous forme de sels) ou un sel pharmaceutiquement acceptable d'un tel composé.

En ce qui concerne les produits, les médicaments et les compositions pharmaceutiques précités, les préférences décrites pour les composés de formule générale **(I)** s'appliquent *mutatis mutandis.*

Les compositions pharmaceutiques contenant un composé de l'invention peuvent être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Conformément à l'invention, on peut préparer les composés de formule générale **(I)** par les procédés décrits ci-après.

### PREPARATION DES COMPOSES DE L'INVENTION :

Deux voies de synthèse peuvent être employées pour préparer les composés de formule générale **(I),** dans laquelle R¹, R² et R³ ont la signification indiquée précédemment. Ces voies de synthèse sont résumées dans le schéma 1 ci-dessous. Selon le caractère labile ou non du radical R¹, l'homme du métier choisira plutôt l'une ou l'autre voie de synthèse.

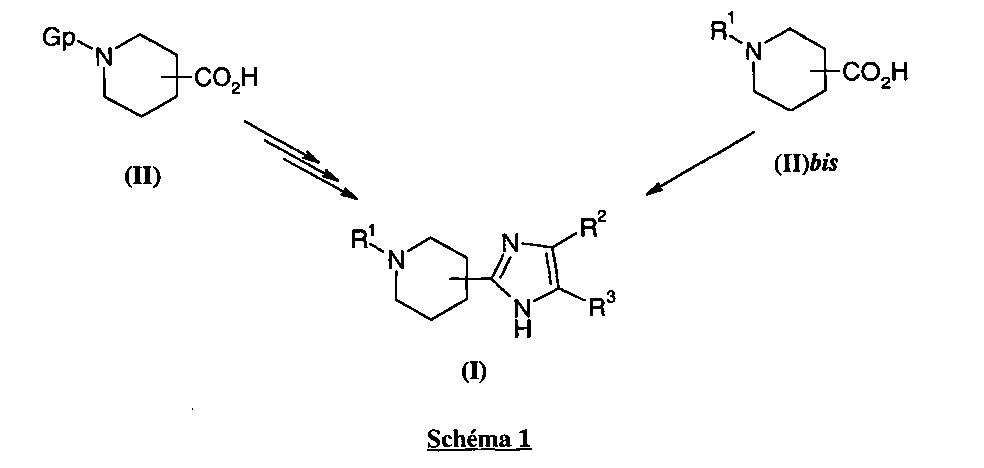

### VOIE 1 : synthèse à partir des composés de formule générale (II) :

Les composés de formule générale **(I),** dans laquelle R¹, R² et R³ ont la signification indiquée précédemment, peuvent être préparés par la voie de synthèse générale illustrée ci-dessous (schéma 2) à partir des intermédiaires de formule générale **(II)** dans laquelle Gp est un groupe protecteur pour une fonction amine (par exemple un groupe protecteur de type carbamate ou tout autre groupe protecteur que l'homme du métier jugera adapté, et notamment ceux cités dans : *Protective groups in organic synthesis*, 2nd ed., (John Wiley & Sons Inc., 1991)). L'acide de formule générale **(II)** est condensé sur l'α-halogénocétone de formule générale **(III)** puis le composé protégé de formule générale **(IV)** est déprotégé pour donner le composé de formule générale **(V)** avant d'être fonctionnalisé de façon adéquate pour donner le composé de formule générale **(I)** (les deux dernières étapes étant inutiles dans le cas particulier où R¹ = Gp).

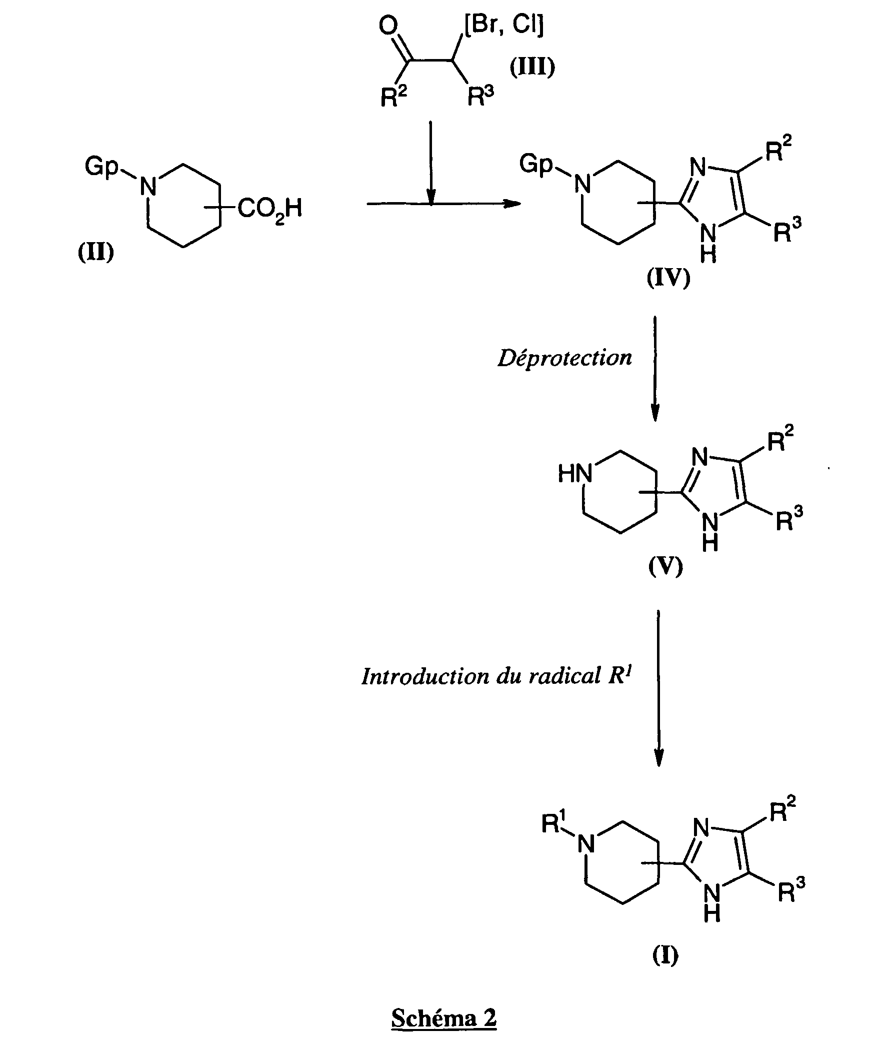

### Préparation des composés de formule générale (II)

Les acides de formule générale **(II)** sont commerciaux ou peuvent être facilement obtenus à partir de composés commerciaux selon des méthodes de synthèse organique classiques bien connues de l'homme du métier.

### Préparation des composés de formule générale (III)

Les α-halogénocétones de formule générale **(III)** peuvent être facilement préparées à partir des cétones correspondantes de formule générale **(III.i)** par une réaction d'halogénation (schéma 3) connue de l'homme du métier.

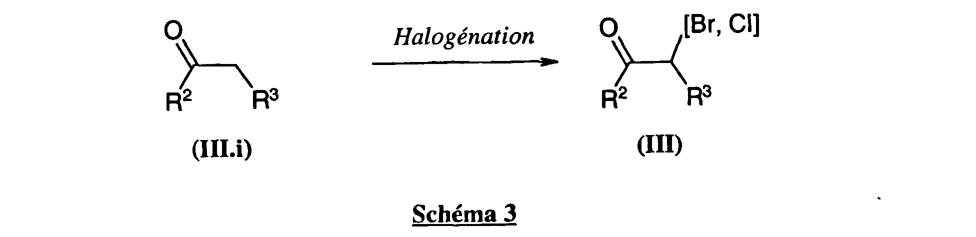

Par exemple, dans le cas d'une bromation, la cétone de formule générale **(III.i)** peut être convertie en l'α-bromocétone correspondante par réaction avec un agent de bromation tel que CuBr₂ (*J. Org. Chem.* (1964), **29,** 3459), du brome (*J. Het. Chem.* (1988), **25,** 337), du N-bromosuccinimide (*J. Amer. Chem. Soc*. (1980), **102,** 2838) en présence d'acide acétique dans un solvant comme l'acétate d'éthyle ou le dichlorométhane, HBr ou Br₂ dans de l'éther ou de l'acide acétique (*Biorg. Med. Chem. Lett.* (1996), **6**(3), 253-258 ; *J. Med. Chem.* (1988), **31**(10), 1910-1918) ou encore à l'aide d'une résine de bromation (*J. Macromol. Sci. Chem.* (1977), **A11,** (3) 507-514).

### Préparation des composés de formule générale (IV) à partir des composés de formule générale (II)

Les composés de formule générale **(IV)** peuvent être obtenus à partir des acides de formule générale **(II)** condensés selon des méthodes connues de l'homme du métier, et par exemple par ajout de carbonate de césium suivi de la condensation avec une α-halogénocétone de formule générale **(III)** suivie de l'addition d'un large excès d'acétate d'ammonium (par exemple 15 ou 20 équivalents par équivalent d'acide de formule générale **(II)).** Cette réaction s'effectue de préférence dans un mélange de xylènes et en chauffant (on peut aussi, le cas échéant, éliminer simultanément l'eau formée au cours de la réaction).

### Préparation des composés de formule générale (V) à partir des composés de formule générale (IV)

La déprotection pour libérer la fonction amine de la pipéridine est effectuée selon des méthodes classiques pour l'homme du métier qui pourra en particulier se référer à l'ouvrage suivant : *Protective groups in organic synthesis*, 2nd ed., (John Wiley & Sons Inc., 1991).

### Préparation des composés de formule générale (I) à partir des composés de formule générale (V)

Selon le type de radical R¹ à introduire sur la fonction amine de la pipéridine, la dernière étape du procédé est choisie de façon adéquate par l'homme du métier, et peut, par exemple, être effectuée selon les méthodes décrites ci-après et résumées dans les schémas 4 à 8.

Lorsque R¹ est un radical alkyle, cycloalkylalkyle, aralkyle ou haloalkyle, les composés de formule générale **(I)** peuvent être obtenus, schéma 4, par une réaction de substitution nucléophile du composé de formule générale **(VI),** dans laquelle Δ représente un radical alkyle, cycloalkylalkyle, aralkyle ou haloalkyle, sur la fonction amine du groupe pipéridyle du composé de formule générale **(V).**

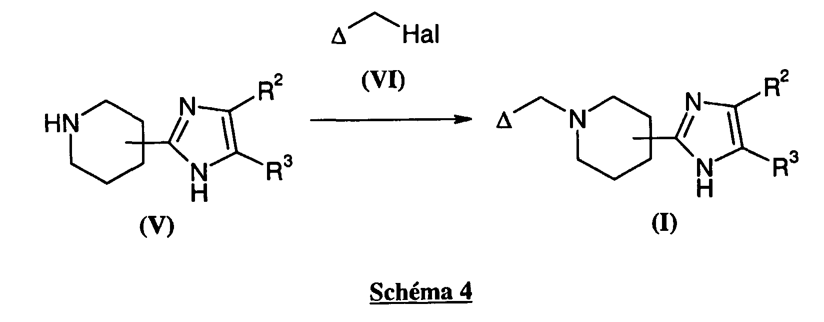

Lorsque R¹ est un radical cycloalkyle, les composés de formule générale **(I)** peuvent être obtenus, schéma 5, par une réaction de condensation des composés de formule générale **(V)** avec les cycloalkylcétones de formule générale **(VII),** dans lesquelles n représente un entier de 0 à 4, en présence d'un agent réducteur comme le triacétoxyborohydrure de sodium ou le borohydrure de sodium dans un alcool aliphatique inférieur comme le méthanol et éventuellement en présence de tamis moléculaires à température ambiante.

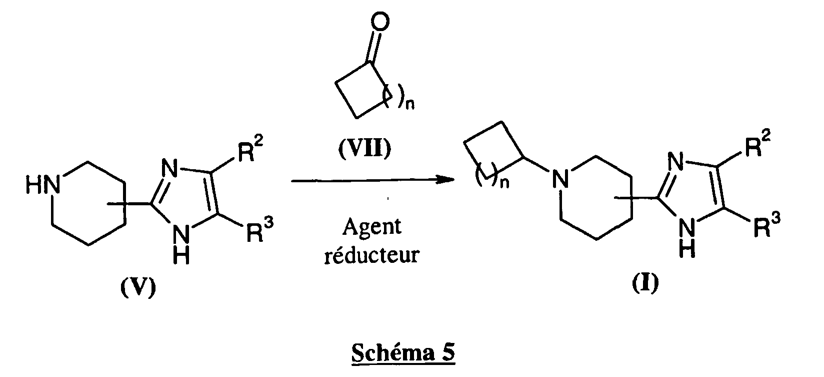

Lorsque R¹ est un radical alkylcarbonyle ou aralkylcarbonyle, les composés de formule générale **(I)** peuvent être obtenus, schéma 6, par condensation de l'acide de formule générale **(VIII)** (ou du chlorure d'acide correspondant), dans laquelle Δ représente un radical alkyle ou aralkyle, sur la fonction amine du groupe pipéridyle du composé de formule générale **(V)** dans les conditions classiques de la synthèse peptidique.

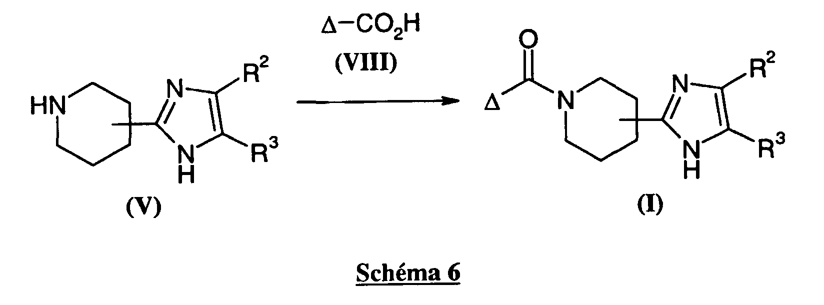

Lorsque R¹ est un radical alkylaminocarbonyle ou aralkylaminocarbonyle, les composés de formule générale **(I)** peuvent être obtenus, schéma 7, par condensation dans un solvant inerte comme le dichlorométhane ou le 1,2-dichloroéthane de l'isocyanate ou l'isothiocyanate de formule générale **(IX),** dans laquelle Δ représente un radical alkyle ou aralkyle, sur la fonction amine du groupe pipéridyle du composé de formule générale **(V)**.

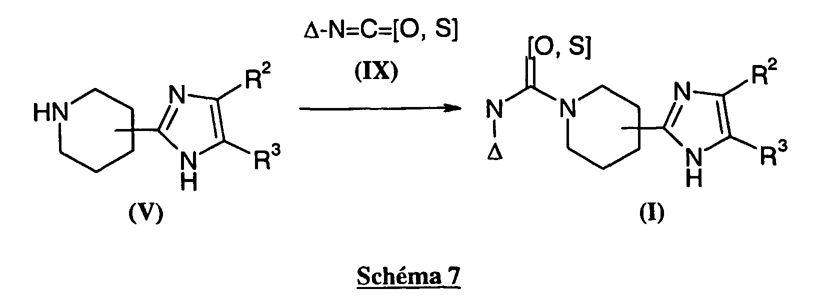

Lorsque R¹ est un radical alkoxycarbonyle ou aralkoxycarbonyle, les composés de formule générale **(I)** peuvent être obtenus, schéma 8, par condensation du composé de formule générale **(X),** dans laquelle Δ représente un radical alkyle ou aralkyle, sur la fonction amine du groupe pipéridyle du composé de formule générale **(V)**. La réaction s'effectue de préférence en présence d'une base comme par exemple NaOH.

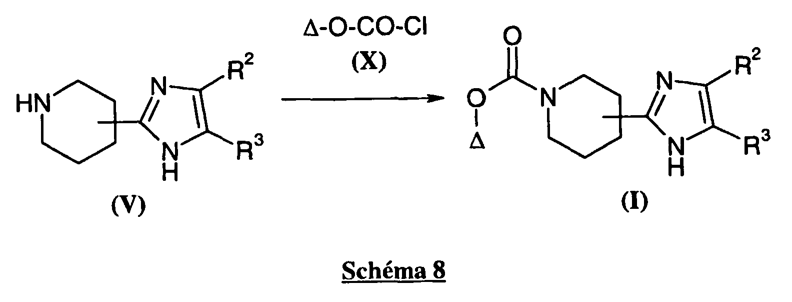

### VOIE 2 : synthèse à partir des composés de formule générale (II)bis :

Certains composés de formule générale **(I)**, dans lesquels R² et R³ ont la signification indiquée précédemment et R¹ est par exemple un radical -CO-O-R⁴ dans lequel R⁴ est tel que défini précédemment, peuvent être préparés en une seule étape par la voie de synthèse générale illustrée ci-dessous (schéma 9) à partir des composés de formule générale **(II)*****bis***. Cette réaction est en tous points similaire à celle qui a déjà été décrite précédemment entre les composés de formule générale **(II)** et les composés de formule générale **(III)**.

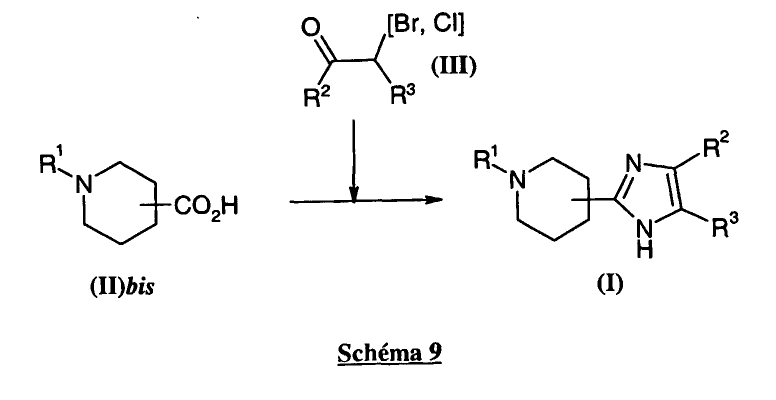

### Préparation des composés de formule générale (II)bis

Les acides de formule générale **(II)*****bis*** sont commerciaux ou peuvent être facilement obtenus à partir de composés commerciaux selon des méthodes de synthèse organique classiques bien connues de l'homme du métier.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES

### Exemple 1 : 4-[4-(4'-bromo-1,1'-biphényl-4-yl)-1H-imidazol-2-yl]pipéridine-1-carboxylate de butyle

### 1.1) acide 1-(butoxycarbonyl)pipéridine-4-carboxylique

Un mélange contenant une solution d'hydroxyde de sodium 1*N* (100 ml) et l'acide pipéridine-4-carboxylique (12,9 g, 0,1 mol) est agité à 23 °C pendant quelques minutes. On ajoute ensuite simultanément goutte à goutte une solution d'hydroxyde de sodium 1*N* (100 ml) et du chloroformate de n-butyle (13,65 g ; 0,1 mol) sans que la température du milieu réactionnel ne dépasse 20 °C. Après 16 h d'agitation à cette température, on ajoute une solution d'acide chlorhydrique 1N jusqu'à obtenir un pH acide. Après extraction avec de l'acétate d'éthyle (2 fois 50 ml), lavage de la phase organique avec une solution saturée en chlorure de sodium et séchage sur sulfate de sodium, les solvants sont évaporés à l'aide d'un évaporateur rotatif. L'huile obtenue cristallise dans un mélange éther isopropylique-isopentane. Le solide est filtré sur fritté et lavé à l'isopentane. On obtient une poudre de couleur blanche avec un rendement de 83%.
MH+ = 230,1.

### 1.2) 2-bromo-1-(4'-bromo-1,1'-biphényl-4-yl)éthanone

De la 4-(4-bromophényl)acétophénone (10 g ; 36,3 mmol) est dissoute dans un mélange méthanol / dichlorométhane (100 / 150 ml). Une résine polymère perbromure d'hydrobromure de pyridine (65 g) est ajoutée et le milieu réactionnel agité à 23 °C pendant 20 h. La résine est récupérée par filtration sur fritté et rincée avec du dichlorométhane. Le filtrat obtenu est évaporé à sec et le solide résultant rincé avec un mélange de solvant dichlorométhane-heptane 1-1 par petits volumes. On obtient une poudre de couleur beige clair avec un rendement de 70%.
RMN H¹(δ ppm, DMSO): 4,96 (s, 2H); 7,69-7,75 (m, 4H); 7,90 (dd, 2H); 8,08 (dd, 2H).

### 1.3) 4-[4-(4'-bromo-1,1'-biphényl-4-yl)-1H-imidazol-2-yl]pipéridine-1-carboxylate de butyle

Un mélange contenant l'acide 1-(butoxycarbonyl)pipéridine-4-carboxylique (préparé à l'étape 1.1 ; 2,29 g ; 0,01 mol) et du carbonate de césium (1,62 g, 0,005 mol) dans 30 ml de méthanol anhydre est agité pendant une heure. Ce mélange est évaporé à sec puis dilué avec 40 ml de diméthylformamide. De la 2-bromo-1-(4'-bromo-1,1'-biphényl-4-yl)éthanone préparée précédemment est ajoutée (4,00 g ; 0,01 mol) puis le mélange résultant agité durant 2 h. Le solvant est évaporé à l'aide d'une pompe à palettes. 50 ml de xylènes sont ajoutés et le bromure de césium est filtré sur fritté. De l'acétate d'ammonium (15,4 g ; 0,2 mol) est alors ajouté et le mélange chauffé au reflux pendant 1 h 30 avant d'être versé dans de l'eau glacée à laquelle on ajoute 80 ml d'acétate d'éthyle. Après décantation, la phase organique est lavée avec une solution saturée en chlorure de sodium. La phase organique est ensuite séchée sur sulfate de magnésium et le solvant évaporé. L'huile obtenue cristallise dans l'éther isopropylique. On filtre sur fritté et recristallise dans 15 ml d'acétate d'isopropyle. Après une nouvelle filtration sur fritté, on rince le solide récupéré à l'éther isopropylique puis à l'isopentane avant de le sécher sous vide. On obtient une poudre de couleur blanche avec un rendement de 17%. Point de fusion : 150-152 °C.

### Exemple 2 : 4-[4-(1,1'-biphényl-4-yl)-1H-imidazol-2-yl]pipéridine-1-carboxylate de tert-butyle

Le protocole expérimental utilisé est identique à celui décrit pour les étapes 1.2 et 1.3 de l'exemple 1, l'acide Boc-isonipécotique (13,8 g, 0,06 mol) remplaçant l'intermédiaire 1.1. Le produit final obtenu est une poudre de couleur beige avec un rendement de 62%. Point de fusion : 120-122 °C.

### Exemple 3 : chlorhydrate de 4-[4-(1,1'-biphényl-4-yl)-1H-imidazol-2-yl]pipéridine

Une suspension contenant le composé de l'exemple 2 (14,9 g, 0,037 mol) dans 100 ml d'acétate d'éthyle est refroidie à environ 5 °C. 100 ml d'une solution d'acide chlorhydrique 3N dans l'acétate d'éthyle sont ajoutés goutte à goutte à cette température. Le mélange réactionnel est ensuite agité environ une heure à 23 °C. Le précipité obtenu est filtré sur fritté puis rincé avec de l'acétate d'éthyle et de l'éther. Après séchage sous vide, on obtient une poudre de couleur beige avec un rendement de 100%. Point de fusion : > 260 °C.

### Exemple 4 : 1-benzyl-4-[4-(1,1'-biphényl-4-yl)-1H-imidazol-2-yl]pipéridine

De la 4-[4-(1,1'-biphényl-4-yl)-1*H*-imidazol-2-yl]pipéridine (préparée à l'exemple 3 ; 0,6 g ; 0,0018 mol) est dissoute dans 20 ml d'acétonitrile. De la triéthylamine (0,6 ml ; 0,0044 mol) puis du bromure de benzyle (0,3 ml ; 0,0024 mol) sont ajoutés. Après agitation pendant 1 h à 23 °C, on évapore les solvants. On ajoute ensuite 50 ml d'eau et 60 ml d'acétate d'éthyle au résidu et extrait la phase aqueuse avec de l'acétate d'éthyle. Les phases organiques regroupées sont lavées avec une solution saturée en chlorure de sodium puis séchées sur sulfate de magnésium. Les solvants sont évaporés et l'huile obtenue est purifiée sur une colonne de silice (éluant: CH₂Cl₂-MeOH/95-5). On obtient une poudre de couleur blanche avec un rendement de 14%. Point de fusion : 110-112 °C.

### Exemple 5 : 1-(cyclohexylméthyl)-4-[4-(4-fluorophényl)-1H-imidazol-2-yl]pipéridine

De la 4-[4-(4-fluorophényl)-1*H*-imidazol-2-yl]pipéridine (préparée selon un mode opératoire analogue à celui de l'exemple 1 ;0,1 g ; 0,0004 mol) est dissoute dans 5 ml de méthanol. Du cyclohexanecarboxaldéhyde (0,06 ml ; 0,0005 mol) est ajouté. Après agitation durant une heure à 23 °C, du triacétoxyborohydrure de sodium (0,17 g ; 0,0008 mol) est ajouté. Après agitation durant deux jours à cette température, on ajoute environ 10 ml d'eau. La phase aqueuse est extraite avec 2 fois 20 ml d'acétate d'éthyle et la phase organique obtenue est lavée avec une solution saturée en chlorure de sodium avant d'être séchée sur sulfate de magnésium et concentrée à l'aide d'un évaporateur rotatif. L'huile résiduelle est cristallisée en utilisant de l'éther. Après filtration sur fritté, les cristaux sont lavés à l'éther et à l'isopentane. On obtient une poudre de couleur beige avec un rendement de 37%. Point de fusion : 220-222 °C.

### Exemple 6 : 4-[4-(1,1'-biphényl-4-yl)-1H-imidazol-2-yl]-N-butylpipéridine-1-carboxamide

De la 4-[4-(1,1'-biphényl-4-yl)-1*H*-imidazol-2-yl]pipéridine (préparée à l'exemple 3 ; 1,12 g ; 0,003 mol) est ajoutée à 20 ml de dichloro-1,2-éthane. A cette suspension à 23 °C, on ajoute de la triéthylamine (0,84 ml ; 0,006 mol) puis du n-butylisocyanate (0,34 ml ; 0,003 mol). Après chauffage à environ 50 °C pendant 30 minutes, on agite le mélange réactionnel à 23 °C pendant deux jours. 20 ml d'eau sont ensuite ajoutés. Le précipité formé est filtré sur fritté puis lavé au dichloro-1,2-éthane et à l'éther. Après séchage sous vide, on obtient une poudre de couleur blanche avec un rendement de 46%. Point de fusion : 99-100 °C.

### Exemple 7 : 4-[4-(1,1'-biphényl-4-yl)-1H-imidazol-2-yl]-N-butylpipéridine-1-carbothioamide

De la 4-[4-(1,1'-biphényl-4-yl)-1*H*-imidazol-2-yl]pipéridine (préparée à l'exemple 3 ; 1,12 g ; 0,003 mol) est ajoutée à 25 ml de dichloro-1,2-éthane. A cette suspension à 23 °C, on ajoute de la triéthylamine (0,84 ml ; 0,006 mol) puis du n-butylisothiocyanate (0,38 ml ; 0,003 mol). Après chauffage à environ 50 °C pendant 30 minutes, on agite le mélange réactionnel à 23 °C pendant deux jours. 20 ml d'eau sont ensuite ajoutés. Le précipité formé est filtré sur fritté puis lavé au dichloro-1,2-éthane et à l'éther. Le solide obtenu est purifié sur une colonne de silice (éluant : CH₂Cl₂-éthanol : 90-10). On obtient une poudre de couleur blanche avec un rendement de 31%. Point de fusion : 102-103 °C.

### Exemple 8 : 4-[4-(1,1'-biphényl-4-yl)-1H-imidazol-2-yl]pipéridine-1-carboxylate d'éthyle

Du chlorhydrate de 4-[4-(1,1'-biphényl-4-yl)-1*H*-imidazol-2-yl]pipéridine (préparée à l'exemple 3 ; 1,12 g ; 0,003 mol) est ajoutée à 30 ml de dichlorométhane. A cette suspension à 23 °C, on ajoute de la triéthylamine (0,83 ml ; 0,006 mol) puis du *n*-éthyl chloroformate (0,28 ml ; 0,003 mol). On agite le mélange réactionnel à 23 °C pendant seize heures. Rajouter ensuite de la triéthylamine (0,42 ml ; 0,003 mol) puis 20 ml d'eau sont ensuite ajoutés. La phase aqueuse est extraite avec 2 fois 20 ml d'acétate d'éthyle et la phase organique obtenue est lavée avec une solution saturée en chlorure de sodium avant d'être séchée sur sulfate de magnésium et concentrée à l'aide d'un évaporateur rotatif. L'huile obtenue est purifié sur une colonne de silice (éluant : Acétate d'éthyle-heptane : 95-5). On obtient une poudre de couleur jaune pâle avec un rendement de 20%. Point de fusion : 71-72 °C.

### Exemple 9 : 4-[4-(1,1'-biphényl-4-yl)-1H-imidazol-2-yl]-1-pentanoylpipéridine

Du chlorhydrate de 4-[4-(1,1'-biphényl-4-yl)-1*H*-imidazol-2-yl]pipéridine (préparé à l'exemple 3 ; 1,12 g ; 0,003 mol) est ajouté à 30 ml de dichlorométhane. A cette suspension à 23 °C, on ajoute de la triéthylamine (0,83 ml ; 0,006 mol) puis du chlorure de pentanoyle (0,36 ml ; 0,003 mol) ; on agite le mélange réactionnel à 23 C pendant 24 heures puis on rajoute de la triéthylamine (0,42 ml ; 0,003 mol). Après une heure d'agitation à 23 °C, on ajoute 20 ml d'eau. Une fois décantée, la phase aqueuse est extraite avec 2 fois 20 ml de dichlorométhane. Les phases organiques sont lavées avec une solution saturée en chlorure de sodium avant d'être séchées sur sulfate de magnésium et concentrées à l'aide d'un évaporateur rotatif. L'huile obtenue est purifiée sur une colonne de silice (éluant : dichlorométhane / éthanol : 100 à 95-5). On obtient une poudre de couleur blanche avec un rendement de 15%. Point de fusion : 215-216 °C.

*Les composés des exemples 10 à 26 sont obtenus selon des procédures analogues à celles décrites pour les exemples 1 à 9 ou ci-dessus dans la partie intitulée « Préparation des composés de formule générale (I)* ».

### Exemple 10 : 4-[4-(4-fluorophényl)-1H-imidazol-2-yl]pipéridine-1-carboxylate de tert-butyle

Base libre. Point de fusion : 180-182 °C.

### Exemple 11 : 4-[4-(4-fluorophényl)-1H-imidazol-2-yl]pipéridine

Chlorhydrate. Point de fusion : > 260 °C.

### Exemple 12 : 1-benzyl-4-[4-(1,1'-biphényl-4-yl)-1H-imidazol-2-yl]pipéridine

Base libre. Point de fusion : 110-112 °C.

### Exemple 13 : 4-[4-(4-tert-butylphényl)-1H-imidazol-2-yl]pipéridine-1-carboxylate de butyle

Base libre. Point de fusion : 98-100 °C.

### Exemple 14 : 1-benzyl-3-[4-(4-fluorophényl)-1H-imidazol-2-yl]pipéridine

Chlorhydrate. MH+ = 336,2.

### Exemple 15 : 3-[4-(4-fluorophényl)-1H-imidazol-2-yl]pipéridine-1-carboxylate de butyle

Base libre. Point de fusion : 96,5 °C.

### Exemple 16 : 2-[4-(4-fluorophényl)-1H-imidazol-2-yl]pipéridine

Chlorhydrate. Point de fusion : 222-223 °C.

### Exemple 17 : 1-benzyl-2-[4-(4-fluorophényl)-1H-imidazol-2-yl]pipéridine

Base libre. Point de fusion : 181,6 °C.

### Exemple 18 : 2-[4-(4-fluorophényl)-1H-imidazol-2-yl]pipéridine-1-carboxylate de butyle

Base libre. Point de fusion : 157,4 °C.

### Exemple 19 : 4-[4-(1,1'-biphényl-4-yl)-1H-imidazol-2-yl]-1-hexylpipéridine

Base libre. Point de fusion : 138-139 °C.

### Exemple 20 : 4-[4-(1,1'-biphényl-4-yl)-1H-imidazol-2-yl]pipéridine-1-carboxylate de butyle

Base libre. Point de fusion : 73-74 °C.

### Exemple 21 : 2-[4-(1,1'-biphényl-4-yl)-1H-imidazol-2-yl]pipéridine

Chlorhydrate. MH+ = 304,2.

### Exemple 22 : 3-[4-(1,1'-biphényl-4-yl)-1H-imidazol-2-yl]pipéridine

Chlorhydrate. Point de fusion : 226-227 °C.

### Exemple 23 : 2-[4-(1,1'-biphényl-4-yl)-1H-imidazol-2-yl]pipéridine-1-carboxylate de butyle

Base libre. Point de fusion : 166-167 °C.

### Exemple 24 : 3-[4-(1,1'-biphényl-4-yl)-1H-imidazol-2-yl]pipéridine-1-carboxylate de butyle

Base libre. Point de fusion : 116-117 °C.

### Exemple 25 : 4-[4-(4-méthoxyphényl)-1H-imidazol-2-yl]pipéridine

Chlorhydrate. Point de fusion : 272-273 °C.

### Exemple 26 : 4-[4-(4-méthoxyphényl)-1H-imidazol-2-yl]pipéridine-1-carboxylate de butyle

Base libre. Point de fusion : 48-50 °C.

### Etude pharmacologique des produits de l'invention

### Test de liaison sur les canaux sodiques de cortex cérébraux de rat

Le test consiste à mesurer l'interaction des composés vis-à-vis de la liaison de la batrachotoxine tritiée sur les canaux sodiques dépendants du voltage selon le protocole décrit par Brown (*J. Neurosci.* (1986), **6**, 2064-2070).

### Préparation des homogénats de cortex cérébraux de rat

Les cortex cérébraux de rat Sprague-Dawley de 230-250 g (Charles River, France) sont prélevés, pesés et homogénéisés à l'aide d'un broyeur Potter muni d'un piston en téflon (10 allers/retours) dans 10 volumes de tampon d'isolement dont la composition est la suivante (sucrose 0,32 M, K₂HPO₄ 5 mM, pH 7,4). L'homogénat subit une première centrifugation à 1000 g pendant 10 minutes. Le surnageant est prélevé et centrifugé à 20000 g pendant 15 minutes. Le culot est repris dans le tampon d'isolement et centrifugé à 20000 g pendant 15 minutes. Le culot obtenu est remis en suspension dans du tampon d'incubation (HEPES 50 mM, KCl 5,4 mM, MgSO₄ 0,8 mM, glucose 5,5 mM, chlorure de choline 130 mM pH 7,4) puis aliquoté et conservé à - 80 °C jusqu'au jour du dosage. La concentration finale en protéines est comprise entre 4 et 8 mg/ml. Le dosage de protéines se fait par un kit commercialisé par BioRad (France).

### Mesure de la liaison de la batrachotoxine tritiée

La réaction de liaison se fait en incubant pendant 1 h 30 à 25 °C 100 µl d'homogénat de cortex de rat contenant 75 µg de protéines avec 100 µl de [³H] batrachotoxine-A 20-alpha benzoate (37,5 Ci/mmol, NEN) à 5 nM (concentration finale), 200 µl de tétrodotoxine à 1 µM (concentration finale) et de venin de scorpion à 40 µg/ml (concentration finale) et 100 µl de tampon d'incubation seul ou en présence des produits à tester aux différentes concentrations. La liaison non spécifique est déterminée en présence de 300 µM de vératridine et la valeur de cette liaison non spécifique est soustraite à toutes les autres valeurs. Les échantillons sont ensuite filtrés à l'aide d'un Brandel (Gaithersburg, Maryland, USA) en utilisant des plaques Unifilter GF/C préincubées avec 0,1 % de polyéthylène imine (20 µl/puits) et rincées 2 fois avec 2 ml de tampon de filtration (HEPES 5 mM, CaCl₂ 1,8 mM, MgSO₄ 0,8 mM, chlorure de choline 130 mM, pH 7,4). Après avoir ajouté 20 µl de Microscint 0®, la radioactivité est comptée à l'aide d'un compteur à scintillation liquide (Topcount, Packard). La mesure est réalisée en duplicat. Les résultats sont exprimés en % de la liaison spécifique de la batrachotoxine tritiée par rapport au témoin.

### Résultats

Les composés des exemples 1 à 9, 12 à 14, 17, 19 à 24 et 26 décrits ci-dessus présentent tous une CI₅₀ inférieure ou égale à 1 µM.

## Revendications

1. Utilisation d'un composé de formule générale **(I)** 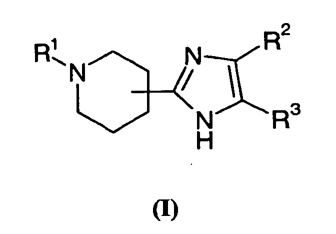 sous forme racémique, d'énantiomère ou toute combinaison de ces formes, dans laquelle :
R¹ représente un atome d'hydrogène ou un radical aralkyle ou cycloalkylalkyle ou encore R¹ représente un radical -X-Y-R⁴ dans lequel le groupe -X-Y- représente -CO-, -CO-O-, -CO-NH- ou -CS-NH- et R⁴ représente un radical alkyle, haloalkyle, cycloalkyle, cycloalkylalkyle, aryle ou aralkyle ;
R² représente un radical phényle substitué par un atome halogène, un radical alkyle ou alkoxy, un radical NR⁵R⁶ ou un radical phényle lui-même éventuellement substitué par un atome halogène ou un radical alkoxy,
R⁵ et R⁶ représentant indépendamment un atome d'hydrogène ou un radical alkyle ou R⁵ et R⁶ formant ensemble avec l'atome d'azote déjà présent un hétérocycle de 5 à 7 chaînons dont les chaînons complémentaires sont choisis parmi -CH₂-, -O-, -S- et -NR⁸-,
R⁸ représentant indépendamment à chaque fois qu'il intervient un atome d'hydrogène ou un radical alkyle, haloalkyle, cycloalkyle, cycloalkylalkyle, aryle ou aralkyle, lesdits radicaux aralkyle ou aryle étant éventuellement substitués de 1 à 4 fois sur le radical aryle par un ou des radicaux choisis parmi un atome halogène et un radical alkyle, hydroxy ou alkoxy ;
R³ représente un atome d'hydrogène,
étant entendu que, dans les définitions qui précèdent :
- chaque radical alkyle ou alkoxy est linéaire ou ramifié et compte de 1 à 12 atomes de carbone ;
- chaque radical haloalkyle est un radical alkyle dont au moins l'un des atomes d'hydrogène est remplacé par un atome halogène
- chaque radical aryle est choisi parmi les radicaux phényle, naphtyle et phénantryle ; et
- chaque radical cycloalkyle compte de 3 à 7 atomes de carbone ;
ou d'un sel pharmaceutiquement acceptable d'un composé de formule générale **(I)** ;
pour préparer un médicament destiné à avoir une activité modulatrice vis-à-vis des canaux sodiques.

2. Utilisation selon la revendication 1, **caractérisée en ce que** le composé de formule générale **(I)** ou son sel pharmaceutiquement acceptable est tel que le cycle pipéridyle des composés de formule générale **(I)** est substitué en position 3 ou 4 par le radical imidazolyle portant les groupes R² et R³.

3. Utilisation selon la revendication 1 ou 2, **caractérisée en ce que** le composé de formule générale **(I)** ou son sel pharmaceutiquement acceptable est tel que R² représente un radical phényle substitué par un radical phényle lui-même éventuellement substitué par un atome halogène ou un radical alkoxy.

4. Utilisation selon la revendication 1, **caractérisée en ce que** le composé de formule générale **(I)** est choisi parmi les composés suivants :
- 4-[4-(4'-bromo-1,1'-biphényl-4-yl)-1H-imidazol-2-yl]pipéridine-1-carboxylate de butyle ;
- 4-[4-(1,1'-biphényl-4-yl)-1*H*-imidazol-2-yl]pipéridine-1-carboxylate de *tert*-butyle ;
- 4-[4-(1,1'-biphényl-4-yl)-1*H*-imidazol-2-yl]pipéridine ;
- 1-benzyl-4-[4-(1,1'-biphényl-4-yl)-1*H*-imidazol-2-yl]pipéridine ;
- 1-(cyclohexylméthyl)-4-[4-(4-fluorophényl)-1*H*-imidazol-2-yl]pipéridine ;
- 4-[4-(1,1'-biphényl-4-yl)-1*H*-imidazol-2-yl]-*N*-butylpipéridine-1-carboxamide ;
- 4-[4-(1,1'-biphényl-4-yl)-1*H*-imidazol-2-yl]-*N*-butylpipéridine-1-carbothioamide ;
- 4-[4-(1,1'-biphényl-4-yl)-1*H*-imidazol-2-yl]pipéridine-1-carboxylate d'éthyle ;
- 4-[4-(1,1'-biphényl-4-yl)-1*H*-imidazol-2-yl]-1-pentanoylpipéridine;
- 4-[4-(4-fluorophényl)-1*H*-imidazol-2-yl]pipéridine-1-carboxylate de *tert*-butyle ;
- 4-[4-(4-fluorophényl)-1*H*-imidazol-2-yl]pipéridine ;
- 1-benzyl-4-[4-(1,1'-biphényl-4-yl)-1*H*-imidazol-2-yl]pipéridine ;
- 4-[4-(4-*tert*-butylphényl)-1*H*-imidazol-2-yl]pipéridine-1-carboxylate de butyle ;
- 1-benzyl-3-[4-(4-fluorophényl)-1*H*-imidazol-2-yl]pipéridine ;
- 3-[4-(4-fluorophényl)-1*H*-imidazol-2-yl]pipéridine-1-carboxylate de butyle ;
- 2-[4-(4-fluorophényl)-1*H*-imidazol-2-yl]pipéridine ;
- 1-benzyl-2-[4-(4-fluorophényl)-1*H*-imidazol-2-yl]pipéridine ;
- 2-[4-(4-fluorophényl)-1*H*-imidazol-2-yl]pipéridine-1-carboxylate de butyle ;
- 4-[4-(1,1'-biphényl-4-yl)-1*H*-imidazol-2-yl]-1-hexylpipéridine ;
- 4-[4-(1,1'-biphényl-4-yl)-1*H*-imidazol-2-yl]pipéridine-1-carboxylate de butyle ;
- 2-[4-(1,1'-biphényl-4-yl)-1*H*-imidazol-2-yl]pipéridine ;
- 3-[4-(1,1'-biphényl-4-yl)-1*H*-imidazol-2-yl]pipéridine ;
- 2-[4-(1,1'-biphényl-4-yl)-1*H*-imidazol-2-yl]pipéridine-1-carboxylate de butyle ;
- 3-[4-(1,1'-biphényl-4-yl)-1*H*-imidazol-2-yl]pipéridine-1-carboxylate de butyle ;
- 4-[4-(4-méthoxyphényl)-1*H*-imidazol-2-yl]pipéridine ;
- 4-[4-(4-méthoxyphényl)-1*H*-imidazol-2-yl]pipéridine-1-carboxylate de butyle ;
et les sels pharmaceutiquement acceptables de ces derniers.

5. Utilisation selon la revendication 4, **caractérisée en ce que** le composé de formule générale **(I)** est choisi parmi les composés suivants :
- 4-[4-(4'-bromo-1,1'-biphényl-4-yl)-1H-imidazol-2-yl]pipéridine-1-carboxylate de butyle ;
- 4-[4-(1,1'-biphényl-4-yl)-1*H*-imidazol-2-yl]pipéridine-1-carboxylate de *tert*-butyle ;
- 4-[4-(1,1'-biphényl-4-yl)-1*H*-imidazol-2-yl]pipéridine ;
- 1-benzyl-4-[4-(1,1'-biphényl-4-yl)-1*H*-imidazol-2-yl]pipéridine ;
- 1-(cyclohexylméthyl)-4-[4-(4-fluorophényl)-1*H*-imidazol-2-yl]pipéridine ;
- 4-[4-(1,1'-biphényl-4-yl)-1*H*-imidazol-2-yl]-*N*-butylpipéridine-1-carboxamide ;
- 4-[4-(1,1'-biphényl-4-yl)-1*H*-imidazol-2-yl]-*N*-butylpipéridine-1-carbothioamide ;
- 4-[4-(1,1'-biphényl-4-yl)-1*H*-imidazol-2-yl]pipéridine-1-carboxylate d'éthyle ;
- 4-[4-(1,1'-biphényl-4-yl)-1*H*-imidazol-2-yl]-1-pentanoylpipéridine;
- 1-benzyl-4-[4-(1,1'-biphényl-4-yl)-1*H*-imidazol-2-yl]pipéridine ;
- 4-[4-(4-*tert*-butylphényl)-1*H*-imidazol-2-yl]pipéridine-1-carboxylate de butyle ;
- 1-benzyl-3-[4-(4-fluorophényl)-1*H*-imidazol-2-yl]pipéridine ;
- 1-benzyl-2-[4-(4-fluorophényl)-1*H*-imidazol-2-yl]pipéridine ;
- 4-[4-(1,1'-biphényl-4-yl)-1*H*-imidazol-2-yl]-1-hexylpipéridine ;
- 4-[4-(1,1'-biphényl-4-yl)-1*H*-imidazol-2-yl]pipéridine-1-carboxylate de butyle ;
- 2-[4-(1,1'-biphényl-4-yl)-1*H*-imidazol-2-yl]pipéridine ;
- 3-[4-(1,1'-biphényl-4-yl)-1*H*-imidazol-2-yl]pipéridine ;
- 2-[4-(1,1'-biphényl-4-yl)-1*H*-imidazol-2-yl]pipéridine-1-carboxylate de butyle ;
- 3-[4-(1,1'-biphényl-4-yl)-1*H*-imidazol-2-yl]pipéridine-1-carboxylate de butyle ;
- 4-[4-(4-méthoxyphényl)-1*H*-imidazol-2-yl]pipéridine-1-carboxylate de butyle ;
et les sels pharmaceutiquement acceptables de ces derniers.

6. Utilisation d'un composé de formule générale **(I)** telle que définie dans l'une des revendications 1 à 5 ou d'un sel pharmaceutiquement acceptable d'un tel composé pour préparer un médicament destiné à traiter la douleur.

7. Utilisation selon la revendication 6, **caractérisé en ce que** le médicament préparé est destiné à traiter les douleurs neuropathiques et la migraine.

8. A titre de médicament, un composé de formule générale **(I)**_{**M**} 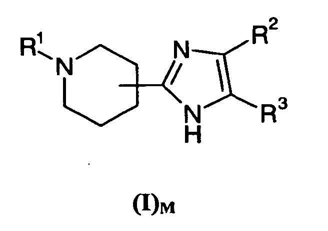 sous forme racémique, d'énantiomère ou toute combinaison de ces formes, dans laquelle :
R¹ représente un radical aralkyle ou cycloalkylalkyle ou encore R¹ représente un radical -X-Y-R⁴ dans lequel le groupe -X-Y- représente -CO-, -CO-O-, -CO-NH- ou -CS-NH- et R⁴ représente un radical alkyle, haloalkyle, cycloalkyle, cycloalkylalkyle, aryle ou aralkyle ;
R² représente un radical phényle substitué par un atome halogène, un radical alkyle ou alkoxy, un radical NR⁵R⁶ ou un radical phényle lui-même éventuellement substitué par un atome halogène ou un radical alkoxy,
R⁵ et R⁶ représentant indépendamment un atome d'hydrogène ou un radical alkyle ou R⁵ et R⁶ formant ensemble avec l'atome d'azote déjà présent un hétérocycle de 5 à 7 chaînons dont les chaînons complémentaires sont choisis parmi -CH₂-, -O-, -S- et -NR⁸-,
R⁸ représentant indépendamment à chaque fois qu'il intervient un atome d'hydrogène ou un radical alkyle, haloalkyle, cycloalkyle, cycloalkylalkyle, aryle ou aralkyle, lesdits radicaux aralkyle ou aryle étant éventuellement substitués de 1 à 4 fois sur le radical aryle par un ou des radicaux choisis parmi un atome halogène et un radical alkyle, hydroxy ou alkoxy ;
R³ représente un atome d'hydrogène
étant entendu que, dans les définitions qui précèdent :
- chaque radical alkyle ou alkoxy est linéaire ou ramifié et compte de 1 à 12 atomes de carbone ;
- chaque radical haloalkyle est un radical alkyle dont au moins l'un des atomes d'hydrogène est remplacé par un atome halogène
- chaque radical aryle est choisi parmi les radicaux phényle, naphtyle et phénantryle ; et
- chaque radical cycloalkyle compte de 3 à 7 atomes de carbone ;
ou les sels pharmaceutiquement acceptables des composés de formule générale **(I)**_{**M**}**.**

9. Médicament **caractérisé en ce qu'**il s'agit d'un des composés suivants :
- 4-[4-(4'-bromo-1,1'-biphényl-4-yl)-1H-imidazol-2-yl]pipéridine-1-carboxylate de butyle ;
- 4-[4-(1,1'-biphényl-4-yl)-1*H*-imidazol-2-yl]pipéridine-1-carboxylate de *tert*-butyle ;
- 4-[4-(1,1'-biphényl-4-yl)-1*H*-imidazol-2-yl]pipéridine ;
- 1-benzyl-4-[4-(1,1'-biphényl-4-yl)-1*H*-imidazol-2-yl]pipéridine ;
- 1-(cyclohexylméthyl)-4-[4-(4-fluorophényl)-1*H*-imidazol-2-yl]pipéridine ;
- 4-[4-(1,1'-biphényl-4-yl)-1*H*-imidazol-2-yl]-*N*-butylpipéridine-1-carboxamide ;
- 4-[4-(1,1'-biphényl-4-yl)-1*H*-imidazol-2-yl]-*N*-butylpipéridine-1-carbothioamide ;
- 4-[4-(1,1'-biphényl-4-yl)-1*H*-imidazol-2-yl]pipéridine-1-carboxylate d'éthyle ;
- 4-[4-(1,1'-biphényl-4-yl)-1*H*-imidazol-2-yl]-1-pentanoylpipéridine ;
- 4-[4-(4-fluorophényl)-1*H*-imidazol-2-yl]pipéridine-1-carboxylate de *tert*-butyle ;
- 4-[4-(4-fluorophényl)-1*H*-imidazol-2-yl]pipéridine ;
- 1-benzyl-4-[4-(1,1'-biphényl-4-yl)-1*H*-imidazol-2-yl]pipéridine ;
- 4-[4-(4-*tert*-butylphényl)-1*H*-imidazol-2-yl]pipéridine-1-carboxylate de butyle ;
- 1-benzyl-3-[4-(4-fluorophényl)-1*H*-imidazol-2-yl]pipéridine ;
- 3-[4-(4-fluorophényl)-1*H*-imidazol-2-yl]pipéridine-1-carboxylate de butyle ;
- 2-[4-(4-fluorophényl)-1*H*-imidazol-2-yl]pipéridine ;
- 1-benzyl-2-[4-(4-fluorophényl)-1*H*-imidazol-2-yl]pipéridine ;
- 2-[4-(4-fluorophényl)-1*H*-imidazol-2-yl]pipéridine-1-carboxylate de butyle ;
- 4-[4-(1,1'-biphényl-4-yl)-1*H*-imidazol-2-yl]-1-hexylpipéridine ;
- 4-[4-(1,1'-biphényl-4-yl)-1*H*-imidazol-2-yl]pipéridine-1-carboxylate de butyle ;
- 2-[4-(1,1'-biphényl-4-yl)-1*H*-imidazol-2-yl]pipéridine ;
- 3-[4-(1,1'-biphényl-4-yl)-1*H*-imidazol-2-yl]pipéridine ;
- 2-[4-(1,1'-biphényl-4-yl)-1*H*-imidazol-2-yl]pipéridine-1-carboxylate de butyle ;
- 3-[4-(1,1'-biphényl-4-yl)-1*H*-imidazol-2-yl]pipéridine-1-carboxylate de butyle ;
- 4-[4-(4-méthoxyphényl)-1*H*-imidazol-2-yl]pipéridine ;
- 4-[4-(4-méthoxyphényl)-1*H*-imidazol-2-yl]pipéridine-1-carboxylate de butyle ;
ou d'un sel pharmaceutiquement acceptable d'un de ces derniers.

10. Produit **caractérisé en ce qu'**il s'agit d'un composé de formule générale **(I)**_{**M**} telle que définie dans la revendication 8 ou d'un sel d'un tel composé.

11. Produit **caractérisé en ce qu'**il s'agit de l'un des composés suivants :
- 4-[4-(4'-bromo-1,1'-biphényl-4-yl)-1H-imidazol-2-yl]pipéridine-1-carboxylate de butyle ;
- 4-[4-(1,1'-biphényl-4-yl)-1*H*-imidazol-2-yl]pipéridine-1-carboxylate de *tert*-butyle ;
- 4-[4-(1,1'-biphényl-4-yl)-1*H*-imidazol-2-yl]pipéridine ;
- 1-benzyl-4-[4-(1,1'-biphényl-4-yl)-1*H*-imidazol-2-yl]pipéridine ;
- 1-(cyclohexylméthyl)-4-[4-(4-fluorophényl)-1*H*-imidazol-2-yl]pipéridine ;
- 4-[4-(1,1'-biphényl-4-yl)-1*H*-imidazol-2-yl]-*N*-butylpipéridine-1-carboxamide ;
- 4-[4-(1,1'-biphényl-4-yl)-1*H*-imidazol-2-yl]-*N*-butylpipéridine-1-carbothioamide ;
- 4-[4-(1,1'-biphényl-4-yl)-1*H*-imidazol-2-yl]pipéridine-1-carboxylate d'éthyle ;
- 4-[4-(1,1'-biphényl-4-yl)-1*H*-imidazol-2-yl]-1-pentanoylpipéridine ;
- 4-[4-(4-fluorophényl)-1*H*-imidazol-2-yl]pipéridine-1-carboxylate de *tert*-butyle ;
- 4-[4-(4-fluorophényl)-1*H*-imidazol-2-yl]pipéridine ;
- 1-benzyl-4-[4-(1,1'-biphényl-4-yl)-1*H*-imidazol-2-yl]pipéridine ;
- 4-[4-(4-*tert*-butylphényl)-1*H*-imidazol-2-yl]pipéridine-1-carboxylate de butyle ;
- 1-benzyl-3-[4-(4-fluorophényl)-1*H*-imidazol-2-yl]pipéridine ;
- 3-[4-(4-fluorophényl)-1*H*-imidazol-2-yl]pipéridine-1-carboxylate de butyle ;
- 2-[4-(4-fluorophényl)-1*H*-imidazol-2-yl]pipéridine ;
- 1-benzyl-2-[4-(4-fluorophényl)-1*H*-imidazol-2-yl]pipéridine ;
- 2-[4-(4-fluorophényl)-1*H*-imidazol-2-yl]pipéridine-1-carboxylate de butyle ;
- 4-[4-(1,1'-biphényl-4-yl)-1*H*-imidazol-2-yl]-1-hexylpipéridine ;
- 4-[4-(1,1'-biphényl-4-yl)-1*H*-imidazol-2-yl]pipéridine-1-carboxylate de butyle ;
- 2-[4-(1,1'-biphényl-4-yl)-1*H*-imidazol-2-yl]pipéridine ;
- 3-[4-(1,1'-biphényl-4-yl)-1*H*-imidazol-2-yl]pipéridine ;
- 2-[4-(1,1'-biphényl-4-yl)-1*H*-imidazol-2-yl]pipéridine-1-carboxylate de butyle ;
- 3-[4-(1,1'-biphényl-4-yl)-1*H*-imidazol-2-yl]pipéridine-1-carboxylate de butyle ;
- 4-[4-(4-méthoxyphényl)-1*H*-imidazol-2-yl]pipéridine ;
- 4-[4-(4-méthoxyphényl)-1*H*-imidazol-2-yl]pipéridine-1-carboxylate de butyle ;
ou d'un sel d'un de ces derniers.

12. Composition pharmaceutique comprenant, à titre de principe actif, au moins un composé de formule générale **(I)**_{**M**} telle que définie dans la revendication 8 ou un sel pharmaceutiquement acceptable d'un tel composé.

13. Composition pharmaceutique comprenant, à titre de principe actif, au moins l'un des composés suivants :
- 4-[4-(4'-bromo-1,1'-biphényl-4-yl)-1H-imidazol-2-yl]pipéridine-1-carboxylate de butyle ;
- 4-[4-(1,1'-biphényl-4-yl)-1*H*-imidazol-2-yl]pipéridine-1-carboxylate de *tert*-butyle ;
- 4-[4-(1,1'-biphényl-4-yl)-1*H*-imidazol-2-yl]pipéridine ;
- 1-benzyl-4-[4-(1,1'-biphényl-4-yl)-1*H*-imidazol-2-yl]pipéridine ;
- 1-(cyclohexylméthyl)-4-[4-(4-fluorophényl)-1*H*-imidazol-2-yl]pipéridine ;
- 4-[4-(1,1'-biphényl-4-yl)-1*H*-imidazol-2-yl]-*N*-butylpipéridine-1-carboxamide ;
- 4-[4-(1,1'-biphényl-4-yl)-1*H*-imidazol-2-yl]-*N*-butylpipéridine-1-carbothioamide ;
- 4-[4-(1,1'-biphényl-4-yl)-1*H*-imidazol-2-yl]pipéridine-1-carboxylate d'éthyle ;
- 4-[4-(1,1'-biphényl-4-yl)-1*H*-imidazol-2-yl]-1-pentanoylpipéridine ;
- 4-[4-(4-fluorophényl)-1*H*-imidazol-2-yl]pipéridine-1-carboxylate de *tert*-butyle ;
- 4-[4-(4-fluorophényl)-1*H*-imidazol-2-yl]pipéridine ;
- 1-benzyl-4-[4-(1,1'-biphényl-4-yl)-1*H*-imidazol-2-yl]pipéridine ;
- 4-[4-(4-*tert*-butylphényl)-1*H*-imidazol-2-yl]pipéridine-1-carboxylate de butyle ;
- 1-benzyl-3-[4-(4-fluorophényl)-1*H*-imidazol-2-yl]pipéridine ;
- 3-[4-(4-fluorophényl)-1*H*-imidazol-2-yl]pipéridine-1-carboxylate de butyle ;
- 2-[4-(4-fluorophényl)-1*H*-imidazol-2-yl]pipéridine ;
- 1-benzyl-2-[4-(4-fluorophényl)-1*H*-imidazol-2-yl]pipéridine ;
- 2-[4-(4-fluorophényl)-1*H*-imidazol-2-yl]pipéridine-1-carboxylate de butyle ;
- 4-[4-(1,1'-biphényl-4-yl)-1*H*-imidazol-2-yl]-1-hexylpipéridine ;
- 4-[4-(1,1'-biphényl-4-yl)-1*H*-imidazol-2-yl]pipéridine-1-carboxylate de butyle ;
- 2-[4-(1,1'-biphényl-4-yl)-1*H*-imidazol-2-yl]pipéridine ;
- 3-[4-(1,1'-biphényl-4-yl)-1*H*-imidazol-2-yl]pipéridine ;
- 2-[4-(1,1'-biphényl-4-yl)-1*H*-imidazol-2-yl]pipéridine-1-carboxylate de butyle ;
- 3-[4-(1,1'-biphényl-4-yl)-1*H*-imidazol-2-yl]pipéridine-1-carboxylate de butyle ;
- 4-[4-(4-méthoxyphényl)-1*H*-imidazol-2-yl]pipéridine ;
- 4-[4-(4-méthoxyphényl)-1*H*-imidazol-2-yl]pipéridine-1-carboxylate de butyle ;
ou un sel pharmaceutiquement acceptables d'un de ces derniers.

## Patentansprüche

1. Verwendung einer Verbindung der allgemeinen Formel (I) 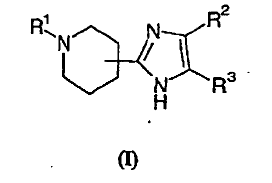 in racemischer Form, enantiomerer Form oder jeder Kombination dieser Formen, worin
R¹ für ein Wasserstoffatom oder einen Aralkyl- oder Cycloalkylalkylrest steht oder ferner R¹ für einen Rest -X-Y-R⁴ steht, worin die Gruppe -X-Y- für -CO-, -CO-O-, -CO-NH- oder -CS-NH- steht, und R⁴ für einen Alkyl-, Halogenalkyl-, Cycloalkyl-, Cycloalkylalkyl-, Aryl- oder Aralkylrest steht;
R² für einen Phenylrest steht, der mit einem Halogenatom, einem Alkyl- oder Alkoxyrest, einem Rest NR⁵R⁶ oder einem Phenylrest, der selbst gegebenenfalls mit einem Halogenatom oder einem Alkoxyrest substituiert ist, substituiert ist,
wobei R⁵ und R⁶ unabhängig für ein Wasserstoffatom oder einen Alkylrest stehen oder R⁵ und R⁶ zusammen mit dem Stickstoffatom, das schon vorliegt, einen Heterocyclus mit 5 bis 7 Ringgliedern bilden, wobei die komplementären Ringglieder aus -CH₂-, -O-, -S- und -NR⁸- ausgewählt sind,
wobei R⁸ unabhängig bei jedem Auftreten für ein Wasserstoffatom oder einen Alkyl-, Halogenalkyl-, Cycloalkyl-, Cycloalkylalkyl-, Aryl- oder Aralkylrest steht, wobei diese Aralkyl- oder Arylreste gegebenenfalls ein- bis viermal am Arylrest mit einem Rest oder Resten, ausgewählt aus einem Halogenatom und einem Alkyl-, Hydroxy- oder Alkoxyrest, substituiert sind;
R³ für ein Wasserstoffatom steht,
wobei in den vorhergehenden Definitionen:
- jeder Alkyl- oder Alkoxyrest linear oder verzweigt ist und 1 bis 12 Kohlenstoffatome zählt;
- jeder Halogenalkylrest einen Alkylrest darstellt, bei dem mindestens eines der Wasserstoffatome durch ein Halogenatom ersetzt ist,
- jeder Arylrest aus den Resten Phenyl, Naphthyl und Phenanthryl ausgewählt ist; und
- jeder Cycloalkylrest 3 bis 7 Kohlenstoffatome zählt;
oder eines pharmazeutisch annehmbaren Salzes einer Verbindung der allgemeinen Formel (I);
zum Herstellen eines Medikaments, das dafür bestimmt ist, eine modulierende Aktivität gegenüber Natriumkanälen aufzuweisen.

2. Verwendung gemäß Anspruch 1, **dadurch gekennzeichnet, dass** die Verbindung der allgemeinen Formel (I) oder deren pharmazeutisch annehmbares Salz derart ist, dass der Piperidylcyclus der Verbindungen der allgemeinen Formel (I) an Position 3 oder 4 mit dem Imidazolylrest, der die Gruppen R² und R³ trägt, substituiert ist.

3. Verwendung gemäß Anspruch 1 oder 2, **dadurch gekennzeichnet, dass** die Verbindung der allgemeinen Formel (I) oder deren pharmazeutisch annehmbares Salz derart ist, dass R² für einen Phenylrest steht, welcher mit einem Phenylrest substituiert ist, der selbst gegebenenfalls mit einem Halogenatom oder einem Alkoxyrest substituiert ist.

4. Verwendung gemäß Anspruch 1, **dadurch gekennzeichnet, dass** die Verbindung der allgemeinen Formel (I) aus den folgenden Verbindungen ausgewählt ist:
- 4-[4-(4'-Brom-1,1'-biphenyl-4-yl)-1*H*-imidazol-2-yl]-piperidin-1-carbonsäurebutylester;
- 4-[4-(1,1'-Biphenyl-4-yl)-1*H*-imidazol-2-yl]-piperidin-1-carbonsäure-*tert*-butylester;
- 4-[4-(1,1'-Biphenyl-4-yl)-1*H*-imidazol-2-yl]-piperidin;
- 1-Benzyl-4-[4-(1,1'-biphenyl-4-yl)-1*H*-imidazol-2-yl]-piperidin;
- 1-(Cyclohexylmethyl)-4-[4-(4-fluorphenyl)-1*H*-imidazol-2-yl]-piperidin;
- 4-[4-(1,1'-Biphenyl-4-yl)-1*H*-imidazol-2-yl]-*N*-butylpiperidin-1-carboxamid;
- 4-[4-(1,1'-Biphenyl-4-yl)-1*H*-imidazol-2-yl]-*N*-butylpiperidin-1-carbothioamid;
- 4-[4-(1,1'-Biphenyl-4-yl)-1*H*-imidazol-2-yl]-piperidin-1-carbonsäureethylester;
- 4-[4-(1,1'-Biphenyl-4-yl)-1*H*-imidazol-2-yl]-1-pentanoylpiperidin;
- 4-[4-(4-Fluorphenyl)-1*H*-imidazol-2-yl]-piperidin-1-carbonsäure-*tert*-butylester;
- 4-[4-(4-Fluorphenyl)-1*H*-imidazol-2-yl]-piperidin;
- 1-Benzyl-4-[4-(1,1'-biphenyl-4-yl)-1*H*-imidazol-2-yl]-piperidin;
- 4-[4-(4-*tert*-Butylphenyl)-1*H*-imidazol-2-yl]-piperidin-1-carbonsäurebutylester;
- 1-Benzyl-3-[4-(4-fluorphenyl)-1*H*-imidazol-2-yl]-piperidin;
- 3-[4-(4-Fluorphenyl)-1*H*-imidazol-2-yl]-piperidin-1-carbonsäurebutylester;
- 2-[4-(4-Fluorphenyl)-1*H*-imidazol-2-yl]-piperidin;
- 1-Benzyl-2-[4-(4-fluorphenyl)-1*H*-imidazol-2-yl]-piperidin;
- 2-[4-(4-Fluorphenyl)-1*H*-imidazol-2-yl]-piperidin-1-carbonsäurebutylester;
- 4-[4-(1,1'-Biphenyl-4-yl)-1*H*-imidazol-2yl]-1-hexylpiperidin;
- 4-[4-(1,1'-Biphenyl-4-yl)-1*H*-imidazol-2-yl]-piperidin-1-carbonsäurebutylester;
- 4-[4-(1,1'-Biphenyl-4-yl)-1*H*-imidazol-2-yl]-piperidin
- 4-[4-(1,1'-Biphenyl-4-yl)-1*H*-imidazol-2-yl]-piperidin
- 2-[4-(1,1'-Biphenyl-4-yl)-1*H*-imidazol-2-yl]-piperidin-1-carbonsäurebutylester;
- 3-[4-(1,1'-Biphenyl-4-yl)-1*H*-imidazol-2-yl]-piperidin-1-carbonsäurebutylester;
- 4-[4-(4-Methoxyphenyl)-1*H*-imidazol-2-yl]-piperidin;
- 4-[4-(4-Methoxyphenyl)-1*H*-imidazol-2-yl]-piperidin-1-carbonsäurebutylester;
und den pharmazeutisch annehmbaren Salzen dieser Verbindungen.

5. Verwendung gemäß Anspruch 4, **dadurch gekennzeichnet, dass** die Verbindung der allgemeinen Formel (I) aus den folgenden Verbindungen ausgewählt ist:
- 4-[4-(4'-Brom-1,1'-biphenyl-4-yl)-1*H*-imidazol-2-yl]-piperidin-1-carbonsäurebutylester;
- 4-[4-(1,1'-Biphenyl-4-yl)-1*H*-imidazol-2-yl]-piperidin-1-carbonsäure-*tert*-butylester;
- 4-[4-(1,1'-Biphenyl-4-yl)-1*H*-imidazol-2-yl]-piperidin;
- 1-Benzyl-4-[4-(1,1'-biphenyl-4-yl)-1*H*-imidazol-2-yl]-piperidin;
- 1 -(Cyclohexylmethyl)-4-[4-(4-fluorphenyl)-1*H*-imidazol-2-yl]-piperidin;
- 4-[4-(1,1'-Biphenyl-4-yl)-1*H*-imidazol-2-yl]-*N*-butylpiperidin-1-carboxamid;
- 4-[4-(1,1'-Biphenyl-4-yl)-1*H*-imidazol-2-yl]-*N*-butylpiperidin-1-carbothioamid;
- 4-[4-(1,1'-Biphenyl-4-yl)-1*H*-imidazol-2-yl]-piperidin-1-carbonsäureethylester;
- 4-[4-(1,1'-Biphenyl-4-yl)-1*H*-imidazol-2-yl]-1-pentanoylpiperidin;
- 1-Benzyl-4-[4-(1,1'-biphenyl-4-yl)-1*H*-imidazol-2-yl]-piperidin;
- 4-[4-(4-*tert*-Butylphenyl)-1*H*-imidazol-2-yl]-piperidin-1-carbonsäurebutylester;
- 1-Benzyl-3-[4-(4-fluorphenyl)-1*H*-imidazol-2-yl]-piperidin;
- 1-Benzyl-2-[4-(4-fluorphenyl)-1*H*-imidazol-2-yl]-piperidin;
- 4-[4-(1,1'-Biphenyl-4-yl)-1*H*-imidazol-2yl]-1-hexylpiperidin;
- 4-[4-(1,1'-Biphenyl-4-yl)-1*H*-imidazol-2-yl]-piperidin-1-carbonsäurebutylester;
- 2-[4-(1,1'-Biphenyl-4-yl)-1*H*-imidazol-2-yl]-piperidin;
- 3-[4-(1,1'-Biphenyl-4-yl)-1*H*-imidazol-2-yl]-piperidin;
- 2-[4-(1,1'-Biphenyl-4-yl)-1*H*-imidazol-2-yl]-piperidin-1-carbonsäurebutylester;
- 3-[4-(1,1'-Biphenyl-4-yl)-1*H*-imidazol-2-yl]-piperidin-1-carbonsäurebutylester;
- 4-[4-(4-Methoxyphenyl)-1*H*-imidazol-2-yl]-piperidin-1-carbonsäurebutylester;
und den pharmazeutisch annehmbaren Salzen dieser Verbindungen.

6. Verwendung einer Verbindung der allgemeinen Formel (I), wie in einem der Ansprüche 1 bis 5 definiert, oder eines pharmazeutisch annehmbaren Salzes einer solchen Verbindung zum Herstellen eines Medikaments, das zur Behandlung von Schmerzen bestimmt ist.

7. Verwendung gemäß Anspruch 6, **dadurch gekennzeichnet, dass** das hergestellte Medikament zur Behandlung von neuropathischen Schmerzen und Migräne bestimmt ist.

8. Eine Verbindung der allgemeinen Formel (I)_{M} als Medikament 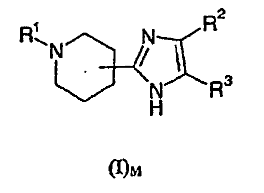 in racemischer Form, enantiomerer Form oder jeder Kombination dieser Formen, worin
R¹ für einen Aralkyl- oder Cycloalkylalkylrest steht oder ferner R¹ für einen Rest -X-Y-R⁴ steht, worin die Gruppe -X-Y- für -CO-, -CO-O-, -CO-NH- oder -CS-NHsteht, und R⁴ für einen Alkyl-, Halogenalkyl-, Cycloalkyl-, Cycloalkylalkyl-, Aryl- oder Aralkylrest steht;
R² für einen Phenylrest steht, der mit einem Halogenatom, einem Alkyl- oder Alk-oxyrest, einem Rest NR⁵R⁶ oder einem Phenylrest, der selbst gegebenenfalls mit einem Halogenatom oder einem Alkoxyrest substituiert ist, substituiert ist,
wobei R⁵ und R⁶ unabhängig für ein Wasserstoffatom oder einen Alkylrest stehen oder R⁵ und R⁶ zusammen mit dem Stickstoffatom, das schon vorliegt, einen Heterocyclus mit 5 bis 7 Ringgliedern bilden, wobei die komplementären Ringglieder aus -CH₂-, -O-, -S- und -NR⁸- ausgewählt sind,
wobei R⁸ unabhängig bei jedem Auftreten für ein Wasserstoffatom oder einen Alkyl-, Halogenalkyl-, Cycloalkyl-, Cycloalkylalkyl-, Aryl- oder Aralkylrest steht, wobei diese Aralkyl- oder Arylreste gegebenenfalls ein- bis viermal am Arylrest mit einem Rest oder Resten, ausgewählt aus einem Halogenatom und einem Alkyl-, Hydroxy- oder Alkoxyrest, substituiert sind;
R³ für ein Wasserstoffatom steht,
wobei in den vorhergehenden Definitionen:
- jeder Alkyl- oder Alkoxyrest linear oder verzweigt ist und 1 bis 12 Kohlenstoffatome zählt;
- jeder Halogenalkylrest einen Alkylrest darstellt, bei dem mindestens eines der Wasserstoffatome durch ein Halogenatom ersetzt ist,
- jeder Arylrest aus den Resten Phenyl, Naphthyl und Phenanthryl ausgewählt ist; und
- jeder Cycloalkylrest 3 bis 7 Kohlenstoffatome zählt;
oder die pharmazeutisch annehmbaren Salze der Verbindungen der allgemeinen Formel (I)_{M}.

9. Medikament, **dadurch gekennzeichnet, dass** es sich um eine der folgenden Verbindungen handelt:
- 4-[4-(4'-Brom-1,1'-biphenyl-4-yl)-1*H*-imidazol-2-yl]-piperidin-1-carbonsäurebutylester;
- 4-[4-(1,1'-Biphenyl-4-yl)-1*H*-imidazol-2-yl]-piperidin-1-carbonsäure-*tert*-butylester;
- 4-[4-(1,1'-Biphenyl-4-yl)-1*H*-imidazol-2-yl]-piperidin;
- 1-Benzyl-4-[4-(1,1'-biphenyl-4-yl)-1*H*-imidazol-2-yl]-piperidin;
- 1-(Cyclohexylmethyl)-4-[4-(4-fluorphenyl)-1*H*-imidazol-2-yl]-piperidin;
- 4-[4-(1,1'-Biphenyl-4-yl)-1*H*-imidazol-2-yl]-*N*-butylpiperidin-1-carboxamid;
- 4-[4-(1,1'-Biphenyl-4-yl)-1*H*-imidazol-2-yl]-*N*-butylpiperidin-1-carbothioamid;
- 4-[4-(1,1'-Biphenyl-4-yl)-1*H*-imidazol-2-yl]-piperidin-1-carbonsäureethylester;
- 4-[4-(1,1'-Biphenyl-4-yl)-1*H*-imidazol-2-yl]-1-pentanoylpiperidin;
- 4-[4-(4-Fluorphenyl)-1*H*-imidazol-2-yl]-piperidin-1-carbonsäure-*tert*-butylester;
- 4-[4-(4-Fluorphenyl)-1*H*-imidazol-2-yl]-piperidin;
- 1 -Benzyl-4-[4-(1, 1'-biphenyl-4-yl)-1*H*-imidazol-2-yl]-piperidin;
- 4-[4-(4-*tert*-Butylphenyl)-1*H*-imidazol-2-yl]-piperidin-1-carbonsäurebutylester;
- 1-Benzyl-3-[4-(4-fluorphenyl)-1*H*-imidazol-2-yl]-piperidin;
- 3-[4-(4-Fluorphenyl)-1*H*-imidazol-2-yl]-piperidin-1-carbonsäurebutylester;
- 2-[4-(4-Fluorphenyl)-1*H*-imidazol-2-yl]-piperidin;
- 1-Benzyl-2-[4-(4-fluorphenyl)-1*H*-imidazol-2-yl]-piperidin;
- 2-[4-(4-Fluorphenyl)-1*H*-imidazol-2-yl]-piperidin-1-carbonsäurebutylester;
- 4-[4-(1,1'-Biphenyl-4-yl)-1*H*-imidazol-2yl]-1-hexylpiperidin;
- 4-[4-(1,1'-Biphenyl-4-yl)-1*H*-imidazol-2-yl]-piperidin-1-carbonsäurebutylester;
- 2-[4-(1,1'-Biphenyl-4-yl)-1*H*-imidazol-2-yl]-piperidin;
- 3-[4-(1,1'-Biphenyl-4-yl)-1*H*-imidazol-2-yl]-piperidin;
- 2-[4-(1,1'-Biphenyl-4-yl)-1*H*-imidazol-2-yl]-piperidin-1-carbonsäurebutylester;
- 3-[4-(1,1'-Biphenyl-4-yl)-1*H*-imidazol-2-yl]-piperidin-1-carbonsäurebutylester;
- 4-[4-(4-Methoxyphenyl)-1*H*-imidazol-2-yl]-piperidin;
- 4-[4-(4-Methoxyphenyl)-1*H*-imidazol-2-yl]-piperidin-1-carbonsäurebutylester;
oder ein pharmazeutisch annehmbares Salz einer dieser Verbindungen.

10. Produkt, **dadurch gekennzeichnet, dass** es sich um eine Verbindung der allgemeinen Formel (I)_{M}, wie im Anspruch 8 definiert, oder um ein Salz einer solchen Verbindung handelt.

11. Produkt, **dadurch gekennzeichnet, dass** es sich um eine der folgenden Verbindungen handelt:
- 4-[4-(4'-Brom-1,1'-biphenyl-4-yl)-1*H*-imidazol-2-yl]-piperidin-1-carbonsäurebutylester;
- 4-[4-(1,1'-Biphenyl-4-yl)-1*H*-imidazol-2-yl]-piperidin-1-carbonsäure-*tert*-butylester;
- 4-[4-(1,1'-Biphenyl-4-yl)-1*H*-imidazol-2-yl]-piperidin;
- 1 -Benzyl-4-[4-(1,1'-biphenyl-4-yl)-1*H*-imidazol-2-yl]-piperidin;
- 1-(Cyclohexylmethyl)-4-[4-(4-fluorphenyl)-1*H*-imidazol-2-yl]-piperidin;
- 4-[4-(1,1'-Biphenyl-4-yl)-1*H*-imidazol-2-yl]-*N*-butylpiperidin-1-carboxamid;
- 4-[4-(1,1'-Biphenyl-4-yl)-1*H*-imidazol-2-yl]-*N*-butylpiperidin-1-carbothioamid;
- 4-[4-(1,1'-Biphenyl-4-yl)-1*H*-imidazol-2-yl]-piperidin-1-carbonsäureethylester;
- 4-[4-(1,1'-Biphenyl-4-yl)-1*H*-imidazol-2-yl]-1-pentanoylpiperidin;
- 4-[4-(4-Fluorphenyl)-1*H*-imidazol-2-yl]-piperidin-1-carbonsäure-*tert*-butylester;
- 4-[4-(4-Fluorphenyl)-1*H*-imidazol-2-yl]-piperidin;
- 1-Benzyl-4-[4-(1,1'-biphenyl-4-yl)-1*H*-imidazol-2-yl]-piperidin;
- 4-[4-(4-*tert*-Butylphenyl)-1*H*-imidazol-2-yl]-piperidin-1-carbonsäurebutylester;
- 1-Benzyl-3-[4-(4-fluorphenyl)-1*H*-imidazol-2-yl]-piperidin;
- 3-[4-(4-Fluorphenyl)-1*H*-imidazol-2-yl]-piperidin-1-carbonsäurebutylester;
- 2-[4-(4-Fluorphenyl)-1*H*-imidazol-2-yl]-piperidin;
- 1-Benzyl-2-[4-(4-fluorphenyl)-1*H*-imidazol-2-yl]-piperidin;
- 2-[4-(4-Fluorphenyl)-1*H*-imidazol-2-yl]-piperidin-1-carbonsäurebutylester;
- 4-[4-(1,1'-Biphenyl-4-yl)-1*H*-imidazol-2yl]-1-hexylpiperidin;
- 4-[4-(1,1'-Biphenyl-4-yl)-1*H*-imidazol-2-yl]-piperidin-1-carbonsäurebutylester;
- 2-[4-(1,1'-Biphenyl-4-yl)-1*H*-imidazol-2-yl]-piperidin;
- 3-[4-(1,1'-Biphenyl-4-yl)-1*H*-imidazol-2-yl]-piperidin;
- 2-[4-(1,1'-Biphenyl-4-yl)-1*H*-imidazol-2-yl]-piperidin-1-carbonsäurebutylester;
- 3-[4-(1,1'-Biphenyl-4-yl)-1*H*-imidazol-2-yl]-piperidin-1-carbonsäurebutylester;
- 4-[4-(4-Methoxyphenyl)-1*H*-imidazol-2-yl]-piperidin;
- 4-[4-(4-Methoxyphenyl)-1*H*-imidazol-2-yl]-piperidin-1-carbonsäurebutylester;
oder ein Salz einer dieser Verbindungen.

12. Pharmazeutische Zubereitung, umfassend als Wirkstoff mindestens eine Verbindung der allgemeinen Formel (I)_{M}, wie in Anspruch 8 definiert, oder ein pharmazeutisch annehmbares Salz einer solchen Verbindung.

13. Pharmazeutische Zubereitung, umfassend als Wirkstoff mindestens eine der folgenden Verbindungen:
- 4-[4-(4'-Brom-1,1'-biphenyl-4-yl)-1*H*-imidazol-2-yl]-piperidin-1-carbonsäurebutylester;
- 4-[4-(1,1'-Biphenyl-4-yl)-1*H*-imidazol-2-yl]-piperidin-1-carbonsäure-*tert*-butylester;
- 4-[4-(1,1'-Biphenyl-4-yl)-1*H*-imidazol-2-yl]-piperidin;
- 1-Benzyl-4-[4-(1,1'-biphenyl-4-yl)-1*H*-imidazol-2-yl]-piperidin;
- 1-(Cyclohexylmethyl)-4-[4-(4-fluorphenyl)-1*H*-imidazol-2-yl]-piperidin;
- 4-[4-(1,1'-Biphenyl-4-yl)-1*H*-imidazol-2-yl]-*N*-butylpiperidin-1-carboxamid;
- 4-[4-(1,1'-Biphenyl-4-yl)-1*H*-imidazol-2-yl]-*N*-butylpiperidin-1-carbothioamid;
- 4-[4-(1,1'-Biphenyl-4-yl)-1*H*-imidazol-2-yl]-piperidin-1-carbonsäureethylester;
- 4-[4-(1,1'-Biphenyl-4-yl)-1*H*-imidazol-2-yl]-1-pentanoylpiperidin;
- 4-[4-(4-Fluorphenyl)-1*H*-imidazol-2-yl]-piperidin-1-carbonsäure-*tert*-butylester;
- 4-[4-(4-Fluorphenyl)-1*H*-imidazol-2-yl]-piperidin;
- 1-Benzyl-4-[4-(1,1'-biphenyl-4-yl)-1*H*-imidazol-2-yl]-piperidin;
- 4-[4-(4-*tert*-Butylphenyl)-1*H*-imidazol-2-yl]-piperidin-1-carbonsäurebutylester;
- 1-Benzyl-3-[4-(4-fluorphenyl)-1*H*-imidazol-2-yl]-piperidin;
- 3-[4-(4-Fluorphenyl)-1*H*-imidazol-2-yl]-piperidin-1-carbonsäurebutylester;
- 2-[4-(4-Fluorphenyl)-1*H*-imidazol-2-yl]-piperidin;
- 1-Benzyl-2-[4-(4-fluorphenyl)-1*H*-imidazol-2-yl]-piperidin;
- 2-[4-(4-Fluorphenyl)-1*H*-imidazol-2-yl]-piperidin-1-carbonsäurebutylester;
- 4-[4-(1,1'-Biphenyl-4-yl)-1*H*-imidazol-2yl]-1-hexylpiperidin;
- 4-[4-(1,1'-Biphenyl-4-yl)-1*H*-imidazol-2-yl]-piperidin-1-carbonsäurebutylester;
- 2-[4-(1,1'-Biphenyl-4-yl)-1*H*-imidazol-2-yl]-piperidin;
- 3-[4-(1,1'-Biphenyl-4-yl)-1*H*-imidazol-2-yl]-piperidin;
- 2-[4-(1,1'-Biphenyl-4-yl)-1*H*-imidazol-2-yl]-piperidin-1-carbonsäurebutylester;
- 3-[4-(1,1'-Biphenyl-4-yl)-1*H*-imidazol-2-yl]-piperidin-1-carbonsäurebutylester;
- 4-[4-(4-Methoxyphenyl)-1*H*-imidazol-2-yl]-piperidin;
- 4-[4-(4-Methoxyphenyl)-1*H*-imidazol-2-yl]-piperidin-1-carbonsäurebutylester;
oder ein pharmazeutisch annehmbares Salz einer dieser Verbindungen.

## Claims

1. Use of a compound of general formula **(I)** 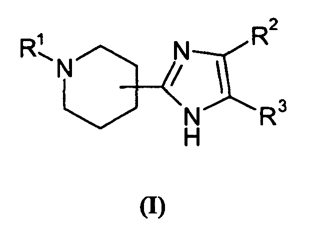 in racemic or enantiomeric form or any combination of these forms, in which:
R¹ represents a hydrogen atom or an aralkyl or cycloalkylalkyl radical or R¹ represents an -X-Y-R⁴ radical in which the -X-Y- group represents -CO-, -CO-O-, -CO-NH- or -CS-NH- and R⁴ represents an alkyl, haloalkyl, cycloalkyl, cycloalkylalkyl, aryl or aralkyl radical;
R² represents a phenyl radical substituted by a halogen atom, an alkyl or alkoxy radical, an NR⁵R⁶ radical or a phenyl radical itself optionally substituted by a halogen atom or an alkoxy radical,
R⁵ and R⁶ representing independently a hydrogen atom or an alkyl radical or R⁵ and R⁶ forming together with the nitrogen atom already present a heterocycle with 5 to 7 members the additional members of which are chosen from -CH₂-, -O-, -S- and -NR⁸-,
R⁸ representing independently each time it is involved a hydrogen atom or an alkyl, haloalkyl, cycloalkyl, cycloalkylalkyl, aryl or aralkyl radical, said aralkyl or aryl radicals being optionally substituted 1 to 4 times on the aryl radical by one or more radicals chosen from a halogen atom and an alkyl, hydroxy or alkoxy radical,
R³ represents a hydrogen atom,
it being understood that, in the preceding definitions:
- each alkyl or alkoxy radical is linear or branched and contains 1 to 12 carbon atoms;
- each haloalkyl radical is an alkyl radical at least one of the hydrogen atoms of which is replaced by a halogen atom
- each aryl radical is chosen from the phenyl, naphthyl and phenantryl radicals; and
- each cycloalkyl radical contains 3 to 7 carbon atoms;
or a pharmaceutically acceptable salt of a compound of general formula **(I);**
for preparing a medicament intended to have a modulatory activity on the sodium channels.

2. Use according to claim 1, **characterized in that** the compound of general formula **(I)** or its pharmaceutically acceptable salt is such that the piperidyl ring of the compounds of general formula **(I)** is substituted in position 3 or 4 by the imidazoyl radical carrying the groups R² and R³.

3. Use according to claim 1 or 2, **characterized in that** the compound of general formula **(I)** or its pharmaceutically acceptable salt is such that R² represents a phenyl radical substituted by a phenyl radical itself optionally substituted by a halogen atom or an alkoxy radical.

4. Use according to claim 1, **characterized in that** the compound of general formula **(I)** is chosen from the following compounds:
- butyl 4-[4-(4'-bromo-1,1'-biphenyl-4-yl)-1H-imidazol-2-yl]piperidine-1-carboxylate;
- *tert*-butyl 4-[4-(1,1'-biphenyl-4-yl)-1*H*-imidazol-2-yl]piperidine-1-carboxylate;
- 4-[4-(1,1'-biphenyl-4-yl)-1*H*-imidazol-2-yl]piperidine;
- 1-benzyl-4-[4-(1,1'-biphenyl-4-yl)-1*H*-imidazol-2-yl]piperidine;
- 1-(cyclohexylmethyl)-4-[4-(4-fluorophenyl)-1*H*-imidazol-2-yl]piperidine;
- 4-[4-(1,1'-biphenyl-4-yl)-1*H*-imidazol-2-yl]-*N*-butylpiperidine-1-carboxamide;
- 4-[4-(1,1'-biphenyl-4-yl)-1*H*-imidazol-2-yl]-*N*-butylpiperidine-1-carbothioamide;
- ethyl 4-[4-(1,1'-biphenyl-4-yl)-1*H*-imidazol-2-yl]piperidine-1-carboxylate;
- 4-[4-(1,1'-biphenyl-4-yl)-1*H*-imidazol-2-yl]-1-pentanoylpiperidine;
- *tert*-butyl 4-[4-(4-fluorophenyl)-1*H*-imidazol-2-yl]piperidine-1-carboxylate;
- 4-[4-(4-fluorophenyl)-1*H*-imidazol-2-yl]piperidine;
- 1-benzyl-4-[4-(1,1'-biphenyl-4-yl)-1*H*-imidazol-2-yl]piperidine;
- butyl 4-[4-(4-*tert*-butylphenyl)-1*H*-imidazol-2-yl]piperidine-1-carboxylate;
- 1-benzyl-3-[4-(4-fluorophenyl)-1*H*-imidazol-2-yl]piperidine;
- butyl 3-[4-(4-fluorophenyl)-1*H*-imidazol-2-yl]piperidine-1-carboxylate;
- 2-[4-(4-fluorophenyl)-1*H*-imidazol-2-yl]piperidine;
- 1-benzyl-2-[4-(4-fluorophenyl)-1*H*-imidazol-2-yl]piperidine;
- butyl 2-[4-(4-fluorophenyl)-1*H*-imidazol-2-yl]piperidine-1-carboxylate;
- 4-[4-(1,1'-biphenyl-4-yl)-1*H*-imidazol-2-yl]-1-hexylpiperidine;
- butyl 4-[4-(1,1'-biphenyl-4-yl)-1*H*-imidazol-2-yl]piperidine-1-carboxylate;
- 2-[4-(1,1'-biphenyl-4-yl)-1*H*-imidazol-2-yl]piperidine;
- 3-[4-(1,1'-biphenyl-4-yl)-1*H*-imidazol-2-yl]piperidine;
- butyl 2-[4-(1,1'-biphenyl-4-yl)-1*H*-imidazol-2-yl]piperidine-1-carboxylate;
- butyl 3-[4-(1,1'-biphenyl-4-yl)-1*H*-imidazol-2-yl]piperidine-1-carboxylate;
- 4-[4-(4-methoxyphenyl)-1*H*-imidazol-2-yl]piperidine;
- butyl 4-[4-(4-methoxyphenyl)-1*H*-imidazol-2-yl]piperidine-1-carboxylate;
and the pharmaceutically acceptable salts of the latter.

5. Use according to claim 4, **characterized in that** the compound of general formula **(I)** is chosen from the following compounds:
- butyl 4-[4-(4'-bromo-1,1'-biphenyl-4-yl)-1H-imidazol-2-yl]piperidine-1-carboxylate;
- *tert*-butyl 4-[4-(1,1'-biphenyl-4-yl)-1*H*-imidazol-2-yl]piperidine-1-carboxylate;
- 4-[4-(1,1'-biphenyl-4-yl)-1*H*-imidazol-2-yl]piperidine;
- 1-benzyl-4-[4-(1,1'-biphenyl-4-yl)-1*H*-imidazol-2-yl]piperidine;
- 1-(cyclohexylmethyl)-4-[4-(4-fluorophenyl)-1*H*-imidazol-2-yl]piperidine;
- 4-[4-(1,1'-biphenyl-4-yl)-1*H*-imidazol-2-yl]-*N*-butylpiperidine-1-carboxamide;
- 4-[4-(1,1'-biphenyl-4-yl)-1*H*-imidazol-2-yl]-*N*-butylpiperidine-1-carbothioamide;
- ethyl 4-[4-(1,1'-biphenyl-4-yl)-1*H*-imidazol-2-yl]piperidine-1-carboxylate;
- 4-[4-(1,1'-biphenyl-4-yl)-1*H*-imidazol-2-yl]-1-pentanoylpiperidine;
- 1-benzyl-4-[4-(1,1'-biphenyl-4-yl)-1*H*-imidazol-2-yl]piperidine;
- butyl 4-[4-(4-*tert*-butylphenyl)-1*H*-imidazol-2-yl]piperidine-1-carboxylate;
- 1-benzyl-3-[4-(4-fluorophenyl)-1*H*-imidazol-2-yl]piperidine;
- 1-benzyl-2-[4-(4-fluorophenyl)-1*H*-imidazol-2-yl]piperidine;
- 4-[4-(1,1'-biphenyl-4-yl)-1*H*-imidazol-2-yl]-1-hexylpiperidine;
- butyl 4-[4-(1,1'-biphenyl-4-yl)-1*H*-imidazol-2-yl]piperidine-1-carboxylate;
- 2-[4-(1,1'-biphenyl-4-yl)-1*H*-imidazol-2-yl]piperidine;
- 3-[4-(1,1'-biphenyl-4-yl)-1*H*-imidazol-2-yl]piperidine;
- butyl 2-[4-(1,1'-biphenyl-4-yl)-1*H*-imidazol-2-yl]piperidine-1-carboxylate;
- butyl 3-[4-(1,1'-biphenyl-4-yl)-1*H*-imidazol-2-yl]piperidine-1-carboxylate;
- butyl 4-[4-(4-methoxyphenyl)-1*H*-imidazol-2-yl]piperidine-1-carboxylate;
and the pharmaceutically acceptable salts of the latter.

6. Use of a compound of general formula **(I)** as defined in one of claims 1 to 5 or of a pharmaceutically acceptable salt of such a compound for preparing a medicament intended to treat pain.

7. Use according to claim 6, **characterized in that** the medicament prepared is intended to treat neuropathic pains and migraine.

8. As a medicament, a compound of general formula **(I)**_{**M**} 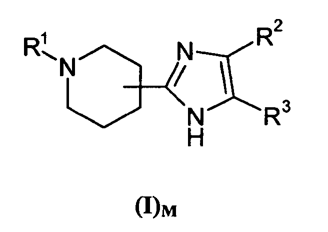 in racemic or enantiomeric form or any combination of these forms, in which:
R¹ represents an aralkyl or cycloalkylalkyl radical or R¹ represents an -X-Y-R⁴ radical in which the -X-Y- group represents a -CO-, -CO-O-, -CO-NH- or -CS-NH- radical and R⁴ represents an alkyl, haloalkyl, cycloalkyl, cycloalkylalkyl, aryl or aralkyl radical;
R² represents a phenyl radical substituted by a halogen atom, an alkyl or alkoxy radical, an NR⁵R⁶ radical or a phenyl radical itself optionally substituted by a halogen atom or an alkoxy radical,
R⁵ and R⁶ representing independently a hydrogen atom or an alkyl radical or R⁵ and R⁶ forming together with the nitrogen atom already present a heterocycle with 5 to 7 members the additional members of which are chosen from -CH₂-, -O-, -S- and -NR⁸-,
R⁸ representing independently each time it is involved a hydrogen atom or an alkyl, haloalkyl, cycloalkyl, cycloalkylalkyl, aryl or aralkyl radical, said aralkyl or aryl radicals being optionally substituted 1 to 4 times on the aryl radical by one or more radicals chosen from a halogen atom and an alkyl, hydroxy or alkoxy radical,
R³ represents a hydrogen atom,
it being understood that, in the preceding definitions:
- each alkyl or alkoxy radical is linear or branched and contains 1 to 12 carbon atoms;
- each haloalkyl radical is an alkyl radical at least one of the hydrogen atoms of which is replaced by a halogen atom
- each aryl radical is chosen from the phenyl, naphthyl and phenantryl radicals; and
- each cycloalkyl radical contains 3 to 7 carbon atoms;
or the pharmaceutically acceptable salts of the compounds of general formula **(I)**_{**M**}**.**

9. Medicament **characterized in that** it is one of the following compounds:
- butyl 4-[4-(4'-bromo-1,1'-biphenyl-4-yl)-1H-imidazol-2-yl]piperidine-1-carboxylate;
- *tert*-butyl 4-[4-(1,1'-biphenyl-4-yl)-1*H*-imidazol-2-yl]piperidine-1-carboxylate;
- 4-[4-(1,1'-biphenyl-4-yl)-1*H*-imidazol-2-yl]piperidine;
- 1-benzyl-4-[4-(1,1'-biphenyl-4-yl)-1*H*-imidazol-2-yl]piperidine;
- 1-(cyclohexylmethyl)-4-[4-(4-fluorophenyl)-1*H*-imidazol-2-yl]piperidine;
- 4-[4-(1,1'-biphenyl-4-yl)-1*H*-imidazol-2-yl]-*N*-butylpiperidine-1-carboxamide;
- 4-[4-(1,1'-biphenyl-4-yl)-1*H*-imidazol-2-yl]-*N*-butylpiperidine-1-carbothioamide;
- ethyl 4-[4-(1,1'-biphenyl-4-yl)-1*H*-imidazol-2-yl]piperidine-1-carboxylate;
- 4-[4-(1,1'-biphenyl-4-yl)-1*H*-imidazol-2-yl]-1-pentanoylpiperidine;
- *tert*-butyl 4-[4-(4-fluorophenyl)-1*H*-imidazol-2-yl]piperidine-1-carboxylate;
- 4-[4-(4-fluorophenyl)-1*H*-imidazol-2-yl]piperidine;
- 1-benzyl-4-[4-(1,1'-biphenyl-4-yl)-1*H*-imidazol-2-yl]piperidine;
- butyl 4-[4-(4-*tert*-butylphenyl)-1*H*-imidazol-2-yl]piperidine-1-carboxylate;
- 1-benzyl-3-[4-(4-fluorophenyl)-1*H*-imidazol-2-yl]piperidine;
- butyl 3-[4-(4-fluorophenyl)-1*H*-imidazol-2-yl]piperidine-1-carboxylate;
- 2-[4-(4-fluorophenyl)-1*H*-imidazol-2-yl]piperidine;
- 1-benzyl-2-[4-(4-fluorophenyl)-1*H*-imidazol-2-yl]piperidine;
- butyl 2-[4-(4-fluorophenyl)-1*H*-imidazol-2-yl]piperidine-1-carboxylate;
- 4-[4-(1,1'-biphenyl-4-yl)-1*H*-imidazol-2-yl]-1-hexylpiperidine;
- butyl 4-[4-(1,1'-biphenyl-4-yl)-1*H*-imidazol-2-yl]piperidine-1-carboxylate;
- 2-[4-(1,1'-biphenyl-4-yl)-1*H*-imidazol-2-yl]piperidine;
- 3-[4-(1,1'-biphenyl-4-yl)-1*H*-imidazol-2-yl]piperidine;
- butyl 2-[4-(1,1'-biphenyl-4-yl)-1*H*-imidazol-2-yl]piperidine-1-carboxylate;
- butyl 3-[4-(1,1'-biphenyl-4-yl)-1*H*-imidazol-2-yl]piperidine-1-carboxylate;
- 4-[4-(4-methoxyphenyl)-1*H*-imidazol-2-yl]piperidine;
- butyl 4-[4-(4-methoxyphenyl)-1*H*-imidazol-2-yl]piperidine-1-carboxylate;
or a pharmaceutically acceptable salt of one of the latter.

10. Product **characterized in that** it is a compound of general formula **(I)**_{**M**} as defined in claim 8 or a salt of such a compound.

11. Product **characterized in that** it is one of the following compounds:
- butyl 4-[4-(4'-bromo-1,1'-biphenyl-4-yl)-1H-imidazol-2-yl]piperidine-1-carboxylate;
- *tert*-butyl 4-[4-(1,1'-biphenyl-4-yl)-1*H*-imidazol-2-yl]piperidine-1-carboxylate;
- 4-[4-(1,1'-biphenyl-4-yl)-1*H*-imidazol-2-yl]piperidine;
- 1-benzyl-4-[4-(1,1'-biphenyl-4-yl)-1*H*-imidazol-2-yl]piperidine;
- 1-(cyclohexylmethyl)-4-[4-(4-fluorophenyl)-1*H*-imidazol-2-yl]piperidine;
- 4-[4-(1,1'-biphenyl-4-yl)-1*H*-imidazol-2-yl]-*N*-butylpiperidine-1-carboxamide;
- 4-[4-(1,1'-biphenyl-4-yl)-1*H*-imidazol-2-yl]-*N*-butylpiperidine-1-carbothioamide;
- ethyl 4-[4-(1,1'-biphenyl-4-yl)-1*H*-imidazol-2-yl]piperidine-1-carboxylate;
- 4-[4-(1,1'-biphenyl-4-yl)-1*H*-imidazol-2-yl]-1-pentanoylpiperidine;
- *tert-*butyl 4-[4-(4-fluorophenyl)-1*H*-imidazol-2-yl]piperidine-1-carboxylate;
- 4-[4-(4-fluorophenyl)-1*H*-imidazol-2-yl]piperidine;
- 1-benzyl-4-[4-(1,1'-biphenyl-4-yl)-1*H*-imidazol-2-yl]piperidine;
- butyl 4-[4-(4-*tert*-butylphenyl)-1*H*-imidazol-2-yl]piperidine-1-carboxylate;
- 1-benzyl-3-[4-(4-fluorophenyl)-1*H*-imidazol-2-yl]piperidine;
- butyl 3-[4-(4-fluorophenyl)-1*H*-imidazol-2-yl]piperidine-1-carboxylate;
- 2-[4-(4-fluorophenyl)-1*H*-imidazol-2-yl]piperidine;
- 1-benzyl-2-[4-(4-fluorophenyl)-1*H*-imidazol-2-yl]piperidine;
- butyl 2-[4-(4-fluorophenyl)-1*H*-imidazol-2-yl]piperidine-1-carboxylate;
- 4-[4-(1,1'-biphenyl-4-yl)-1*H*-imidazol-2-yl]-1-hexylpiperidine;
- butyl 4-[4-(1,1'-biphenyl-4-yl)-1*H*-imidazol-2-yl]piperidine-1-carboxylate;
- 2-[4-(1,1'-biphenyl-4-yl)-1*H*-imidazol-2-yl]piperidine;
- 3-[4-(1,1'-biphenyl-4-yl)-1*H*-imidazol-2-yl]piperidine;
- butyl 2-[4-(1,1'-biphenyl-4-yl)-1*H*-imidazol-2-yl]piperidine-1-carboxylate;
- butyl 3-[4-(1,1'-biphenyl-4-yl)-1*H*-imidazol-2-yl]piperidine-1-carboxylate;
- 4-[4-(4-methoxyphenyl)-1*H*-imidazol-2-yl]piperidine;
- butyl 4-[4-(4-methoxyphenyl)-1*H*-imidazol-2-yl]piperidine-1-carboxylate;
or a salt of one of the latter.

12. Pharmaceutical composition comprising, as active ingredient, at least one compound of general formula **(I)**_{**M**} as defined in claim 8 or a pharmaceutically acceptable salt of such a compound.

13. Pharmaceutical composition comprising, as active ingredient, at least one of the following compounds:
- butyl 4-[4-(4'-bromo-1,1'-biphenyl-4-yl)-1H-imidazol-2-yl]piperidine-1-carboxylate;
- *tert-*butyl 4-[4-(1,1'-biphenyl-4-yl)-1*H*-imidazol-2-yl]piperidine-1-carboxylate;
- 4-[4-(1,1'-biphenyl-4-yl)-1*H*-imidazol-2-yl]piperidine;
- 1-benzyl-4-[4-(1,1'-biphenyl-4-yl)-1*H*-imidazol-2-yl]piperidine;
- 1-(cyclohexylmethyl)-4-[4-(4-fluorophenyl)-1*H*-imidazol-2-yl]piperidine;
- 4-[4-(1,1'-biphenyl-4-yl)-1*H*-imidazol-2-yl]-*N*-butylpiperidine-1-carboxamide;
- 4-[4-(1,1'-biphenyl-4-yl)-1*H*-imidazol-2-yl]-*N*-butylpiperidine-1-carbothioamide;
- ethyl 4-[4-(1,1'-biphenyl-4-yl)-1*H*-imidazol-2-yl]piperidine-1-carboxylate;
- 4-[4-(1,1'-biphenyl-4-yl)-1*H*-imidazol-2-yl]-1-pentanoylpiperidine;
- *tert*-butyl 4-[4-(4-fluorophenyl)-1*H*-imidazol-2-yl]piperidine-1-carboxylate;
- 4-[4-(4-fluorophenyl)-1*H*-imidazol-2-yl]piperidine;
- 1-benzyl-4-[4-(1,1'-biphenyl-4-yl)-1*H*-imidazol-2-yl]piperidine;
- butyl 4-[4-(4-*tert*-butylphenyl)-1*H*-imidazol-2-yl]piperidine-1-carboxylate;
- 1-benzyl-3-[4-(4-fluorophenyl)-1*H*-imidazol-2-yl]piperidine;
- butyl 3-[4-(4-fluorophenyl)-1*H*-imidazol-2-yl]piperidine-1-carboxylate;
- 2-[4-(4-fluorophenyl)-1*H*-imidazol-2-yl]piperidine;
- 1-benzyl-2-[4-(4-fluorophenyl)-1*H*-imidazol-2-yl]piperidine;
- butyl 2-[4-(4-fluorophenyl)-1*H*-imidazol-2-yl]piperidine-1-carboxylate;
- 4-[4-(1,1'-biphenyl-4-yl)-1*H*-imidazol-2-yl]-1-hexylpiperidine;
- butyl 4-[4-(1,1'-biphenyl-4-yl)-1*H*-imidazol-2-yl]piperidine-1-carboxylate;
- 2-[4-(1,1'-biphenyl-4-yl)-1*H*-imidazol-2-yl]piperidine;
- 3-[4-(1,1'-biphenyl-4-yl)-1*H*-imidazol-2-yl]piperidine;
- butyl 2-[4-(1,1'-biphenyl-4-yl)-1*H*-imidazol-2-yl]piperidine-1-carboxylate;
- butyl 3-[4-(1,1'-biphenyl-4-yl)-1*H*-imidazol-2-yl]piperidine-1-carboxylate;
- 4-[4-(4-methoxyphenyl)-1*H*-imidazol-2-yl]piperidine;
- butyl 4-[4-(4-methoxyphenyl)-1*H*-imidazol-2-yl]piperidine-1-carboxylate;
or a pharmaceutically acceptable salt of one of the latter.
